# EUROPEAN PATENT APPLICATION

(11) **EP 4 717 771 A1**
(43) Date of publication of application: **01.04.2026**
(21) Application number: 24202670.6
(22) Date of filing: 25.09.2024
(51) Int. Cl.: C12N 15/113, C12N 9/22

(54) **REDUCING EXPRESSION OF PROTEINASE 3 AUTOANTIGEN IN ANCA-ASSOCIATED VASCULITIS**

(71) Applicant: Charité - Universitätsmedizin Berlin Körperschaft des öffentlichen Rechts, 10117 Berlin (DE); Max-Delbrück-Centrum für Molekulare Medizin in der Helmholtz-Gemeinschaft, 13125 Berlin (DE)
(72) Inventor: KETTRITZ, Ralph, 10785 Berlin (DE); JERKE, Uwe, 13467 Berlin (DE); EULENBERG-GUSTAVUS, Claudia, 16540 Hohen Neuendorf (DE); WAGNER, Dimitrios Laurin, Houston, 77030 (US)
(74) Representative: Moré, Solveig Helga

(57) **Abstract**

The present invention relates to a new therapeutic approach for treatment of ANCA-associated vasculitis (Anti-neutrophil cytoplasmic autoantibody-associated vasculitis, AAV). It provides an agent for reducing expression of proteinase 3 (PR3) in a cell, in particular, a gene editing compound targeting the proteinase 3 gene, an epigenetic editing compound targeting the proteinase 3 gene, or an expression vector encoding an inhibitory oligonucleotide capable of selectively binding to a target nucleic acid sequence in mRNA encoding proteinase 3, e.g., an inhibitory oligonucleotide capable of inducing RNAi or an antisense oligonucleotide. Further, a method of reducing expression of proteinase 3 in a hematopoietic stem cell or cell derived therefrom, e.g., a neutrophil, is provided, as well as such cells having a reduced expression of proteinase 3. The cells or agents can be used for treating a subject having a proteinase 3-ANCA-associated vasculitis.

## Description

The present invention relates to a new therapeutic approach for treatment of ANCA-associated vasculitis (Anti-neutrophil cytoplasmic autoantibody-associated vasculitis, AAV). It provides an agent for reducing expression of proteinase 3 (PR3) in a cell, in particular, a gene editing compound targeting the proteinase 3 gene, an epigenetic editing compound targeting the proteinase 3 gene, or an expression vector encoding an inhibitory oligonucleotide capable of selectively binding to a target nucleic acid sequence in mRNA encoding proteinase 3, e.g., an inhibitory oligonucleotide capable of inducing RNAi or an antisense oligonucleotide. Further, a method of reducing expression of proteinase 3 in a hematopoietic stem cell or cell derived therefrom, e.g., a neutrophil, is provided, as well as such cells having a reduced expression of proteinase 3. The cells or agents can be used for treating a subject having a proteinase 3-ANCA-associated vasculitis.

ANCA-associated vasculitides (AAV) are life-threatening systemic autoimmune diseases. Major clinical AAV entities are granulomatosis with polyangiitis (GPA) and microscopic polyangiitis (MPA) that can affect every organ in the human body, most frequently kidneys and lungs. For unknown reasons, patients lose tolerance to either proteinase 3 (PR3) or myeloperoxidase (MPO) and consequently develop PR3- or MPO-ANCA. ANCA are anti-neutrophil cytoplasmic autoantibodies, which were historically distinguished as p-ANCA or c-ANCA, depending on the perinuclear or cytoplasmic staining detected in indirect immunofluorescence. c-ANCA were found to target proteinase 3, i.e., PR3-ANCA refers to autoantibodies capable of specifically binding the proteinase 3 antigen. Autoantigen-driven adaptive and innate immune responses co-operate in the disease process leading to highly inflammatory, necrotizing small-vessel vasculitis. ANCA-activated neutrophils and monocytes are central to this process, because these cells exclusively express the ANCA autoantigens, are bound and activated by ANCA, and function as important effector cells for vascular inflammation and injuryⁱ. Animal models for MPO-AAV are established but appropriate models for PR3-AAV are lacking¹, presumably because of disease-relevant differences between human and non-human PR3² and CD177³, the PR3-presenting surface-receptor^{4,5} on neutrophils.

Systemic AAV is potentially lethal if untreated. Current treatments include glucocorticoids, cytotoxic drugs, antibody-induced B-cell depletion, and C5a-receptor blocker. Recently, CD19 CAR T-cells⁶ and plasma cell-depleting CD38 antibodies were explored in preclinical AAV models and patients. The goal of these strategies is to suppress the autoimmune responses towards the ANCA autoantigens. The present standard therapy comprises an aggressive immunosuppressive induction therapy followed by a multi-year maintenance therapy. Although immunosuppression effectively reduces AAV activity in the majority of patients, treatment-refractory AAV and treatment-related adverse events prevent better patient outcomes demonstrating the medical need for alternative treatments. According to a meta-analysis of randomized controlled studies, 58% of AAV patients have a relapse after a median of 3.6 years. With every first manifestation and every relapse of AAV, even under immunosuppressive therapy, there is irreversible harm to organs. Further, immunosuppression is linked to significant undesired effects.

In light of this, the inventors addressed the problem of providing an improved treatment for PR3-AAV that overcomes at least some of these disadvantages, for example, that avoids or minimizes relapses and/or does not require a continuous immunosuppressive therapy.

This problem is solved by the present invention, in particular, by the subject-matter of the claims. The invention provides an agent for reducing expression of proteinase 3 in a cell, wherein the agent is
a) a gene editing compound targeting the proteinase 3 gene, or
b) an epigenetic editing compound targeting the proteinase 3 gene, or
c) an expression vector encoding an inhibitory oligonucleotide capable of selectively binding to a target nucleic acid sequence in mRNA encoding proteinase 3, wherein the inhibitory oligonucleotide is selected from the group comprising an RNA molecule capable of inducing RNAi and an antisense oligonucleotide (ASO).

Gene editing provides a novel treatment strategy to ensure long-term improvements for a variety of diseases. Gene editing uses programmable nucleases, such as CRISPR-Cas9, to specifically induce DNA changes at specified sites in the genome of the transfected cells. Induction of DNA-breaks in protein coding genes can force the introduction of insertions and deletions (indels) through error-prone DNA-repair pathways, such as Non-Homologous-End-Joining (NHEJ), compromising protein expression and function. Co-delivery of a nuclease and a DNA repair template is used to exploit homology-directed DNA repair (HDR) to install a desired change, such as the correction of a mutation or insertion of a complete healthy gene fragment. Successful gene editing by either disrupting genes or gene regulatory elements was demonstrated in patients with β-haemoglobinopathies^{7,8}, hereditary angioedema⁹, transthyretin amyloidosis¹⁰ and HIV¹¹, and is currently explored in additional diseases. In December 2023, CRISPR-Cas9 gene editing of human hematopoietic stem and progenitor cells (HSPCs, also designated hematopoietic stem cells herein) was approved for sickle cell disease (SCD) by the U.S. Food and Drug Administration (FDA) and for SCD and β-thalassemia by the European Medicine Agency (EMA). For MPO-associated diseases such as MPO-AAV, an approach comprising modulation of the expression and activity of MPO in undifferentiated bone marrow cells has been suggested ^{28,29}.

The present invention applies this approach to PR3-AAV, and proves the hypothesis that reduced expression of PR3, e.g., due to *ex vivo* CRISPR-Cas9 gene editing, in human CD34+ HSPCs effectively reduces or eliminates the PR3 protein, thereby reducing PR3-ANCA binding to neutrophil-differentiated HSPCs and activation of these cells without compromising defense responses to non-ANCA stimuli.

This approach provides an alternative to immunosuppressive treatments. Thus, durable remission in PR3-AAV patients without continued immunosuppressive treatment is rendered possible. PR3 has also been designated myeloblastin, because it was assumed it played an important role in differentiation of myeloid cells. However, the inventors have surprisingly shown that reduced or abrogated expression of PR3 did not negatively affect neutrophil differentiation of human CD34⁺ HSPCs. Further, in contrast to diseases or disorders where cathepsin C is disturbed, and accordingly, both proteinase 3 and neutrophil elastase function are affected, proteinase 3 deficiency in hematopoietic stem cells does not lead to defective immune responses. Thus, the inventors could show that a therapeutic reduction or abrogation of proteinase 3 is safe in human patients.

The cells targeted with the agent in the present invention preferably are human CD34+ HSPCs. As the present invention leads to a stable modification of the cells, all hematopoietic cells derived from engineered CD34+ HSPC, in particular, neutrophils, are also engineered to have a reduced expression of proteinase 3. The reduction of proteinase 3 is preferably detected in neutrophils derived from CD34+ HSPC modified with the agent of the invention, as neutrophils are the cells most relevant for the development of the disease. Similarly, expression in monocytes should also be reduced.

In the context of the invention, reducing the expression of proteinase 3 in a cell preferably means that the expression of proteinase 3 is reduced by at least 50%, more preferably, by at least 70%, at least 80% or at least 90%. Optimally, expression is substantially or completely abrogated. The reduction in proteinase 3 expression can be detected by various methods, e.g., in cell lysate of neutrophils using an ELISA for proteinase 3, or, preferably, by immunofluorescence of neutrophils, e.g., with flow cytometric measurements, such as described in the experimental part, because that enables an easy quantification of expression.

A particularly relevant method for detecting proteinase 3 expression or reduction of the same is an indirect immunofluorescence assay such as that also used as the clinical standard for detecting proteinase 3-based ANCA. In this assay, neutrophils are permeabilized and the cells are contacted with proteinase 3-based ANCA patient sera that are directly or indirectly labeled with a fluorescent label, e.g., as described in the experimental part below.

The agent is preferably able to reduce expression of proteinase 3 in neutrophils as detected by all of these methods. A detection of reduction in only one of these methods, e.g., in a flow cytometric immunofluorescence assay, is however sufficient. A reduction of expression of proteinase 3 further leads to loss of the proteinase's function, which can be detected and quantified as also shown below.

Accordingly, in a preferred embodiment, the agent of the invention is capable or reducing expression pf PR3 in neutrophils derived from CD34+ HSPC modified with said agent by at least 50%, preferably, by at least 70%, at least 80% or at least 90%, optionally, as detected in a flow cytometric immunofluorescence assay.

In some embodiments, e.g., in preferred embodiments described below, the reduction in proteinase 3 expression on protein level is also reflected in proteinase 3 mRNA levels. This can e.g., be detected by qPCR or other suitable methods for quantifying mRNA.

In one embodiment, no fragment of PR3 is any more produced. As the disease is not mediated by a specific target antigen in proteinase 3, but patients' autoantibodies are polyclonal, it is advantageous if the reduced expression is reduced expression of fragments of proteinase 3 in a neutrophil derived from a modified HSPC that comprise less than 100, optionally, less than 65 consecutive amino acids of the wildtype proteinase 3 sequence. In a preferred embodiment, a frame shift in the codon encoding amino acid 65 of the proteinase 3 sequence has the consequence that at most 64 consecutive amino acids of the wildtype proteinase 3 sequence are expressed, here in combination with a non-proteinase 3 sequence. Such small fragments, or fragments comprising a proteinase 3 sequence and a non-proteinase 3 sequence because of a frameshift are often degraded by the cell and not presented on the cell surface. Thus, they do not lead to an immunologic reactivity with proteinase 3-ANCA.

A modification leading to reduced expression of proteinase 3 can be a knock-out or a knock-down of proteinase 3 expression. It is however important that the genetic modification is stable. Neutrophils have a fast turn-over, so the optimal way of addressing the problem is to genetically modify with the agent of the invention a CD34+ HSPC, which will maintain the modification upon proliferation and differentiation to neutrophils. Therefore, although it is preferred to contact a CD34+ HSPC with the agent of the invention, it is preferred to quantify reduction of expression in neutrophils derived from the HSPC. For this end, they can be compared with neutrophils derived from non-modified HSPC not contacted with the agent of the invention.

A stable modification can be obtained by different agents and approaches:
In a preferred embodiment, the agent is a gene editing compound targeting the proteinase 3 gene. The gene editing compound is selected from
- a gene editor comprising
   i) a ribonucleoprotein complex of a Cas protein and a guide RNA,
   ii) a Zinc finger protein or
   iii) a Transcription activator-like effector nuclease (TALEN), and
- a nucleic acid encoding the gene editor, i.e., a nucleic acid encoding
   i) a ribonucleoprotein complex of a Cas protein and a guide RNA,
   ii) a Zinc finger protein or
   iii) a Transcription activator-like effector nuclease (TALEN).

The preferred gene editor of the invention is a ribonucleoprotein complex of a Cas protein, i.e., it enables CRISPR/Cas-mediated gene editing. This employs regular double-strand cutting nuclease enzymes preferably, but not only, Cas9 (derived from *Streptococcus pyogenes* or functional mutants thereof). Cas12a (derived from *Acidaminococcus sp.* or functional mutants thereof) can also be employed. Other Cas enzymes, e.g., *Staphylococcus aureus* Cas9, *Streptococcus thermophiles* Cas9, or LbCas12a can also be used.

Cas enzymes can also be genetically modified Cas enzymes. A functional mutant of a wildtype enzyme may, e.g., have at least 70%, at least 80% or at least 90% sequence identity to the wildtype enzyme and retains the ability to mediate gene editing, in particular, editing of the targets described herein. They can also be Cas derivative enzymes, including e.g. high fidelity enzymes ¹², base editing enzymes ^{13,14,15}, or prime editors ¹⁶. For example, Cas9 can be Cas9-NG, which recognizes a minimal NG PAM (protospacer adjacent motif). Base editing avoids double strand-breaks and can mediate single nucleotide changes with high precision and low risk. Base editors can e.g. be CBE or ABE (CBE: cytosine base editor, ABE: adenine base editor). In prime editing, new genetic information is written into a target site from a prime editing guide RNA. Prime editing employs a fusion protein consisting of a catalytically impaired Cas endonuclease, in particular, a nickase (e.g., a Cas9 H840A nickase), fused to a reverse transcriptase enzyme (e.g., M-MLV reverse transcriptase) and a prime editing guide RNA (pegRNA), capable of identifying the target site and providing the new genetic information to replace the target DNA nucleotides. It can mediate targeted insertions, deletions, and base-to-base conversions without the need for double strand breaks or donor DNA templates.

Gene editing can also be performed with other programmable nucleases such as TALEN or zinc finger nucleases, including respective derivative enzymes. TALEN (Transcription activator-like effector nucleases) are restriction enzymes that can be engineered to cut specific sequences of DNA. They comprise a TAL effector DNA-binding domain fused to a DNA cleavage domain (a nuclease which cuts DNA strands). Transcription activator-like effectors (TALEs) can be engineered to bind to a target DNA sequence in the proteinase 3 gene, so when combined with a nuclease, DNA can be cut at specific locations in the target gene. Similarly, zinc finger-nucleases are artificial restriction enzymes comprising a zinc finger DNA-binding domain, which is engineered to target a sequence of the proteinase 3 gene, fused to a DNA-cleavage domain. TALEN and Zinc finger nucleases can be very specific.

However, in contrast to these approaches, Cas enzymes do not require engineering of proteins to bind to the desired target sequence but merely provision of a suitable guide RNA, which is easier to provide. Therefore, Cas-mediated gene editing is preferred.

CRISP/Cas-mediated gene editing in the context of the invention is understood to comprise derivative technologies such as base editing or prime editing. Accordingly, the gene editor can also be a base editor or a prime editor.

If the cells of the invention are generated using CRISPR/Cas derivative technologies such as base editor proteins or prime editing, the genetic intervention can also be used to insert a stop codon (or a premature stop codon) in the reading frame, e.g., the early reading frame, or disrupt splice acceptor/donor sites, to prevent generation of functional PR3 protein. The cells of the invention then comprise a stop codon in the reading frame of the proteinase 3 gene, and/or a disrupted splice acceptor/donor site.

The gene editing may also lead to a frameshift, in particular, a frameshift that does not allow for expression, or stable expression of proteinase 3 fragments of more than 100, preferably, more than 64 aa. In this context, an amino acid (sub-) sequence that does not correspond to the wild type proteinase 3 sequence, preferably, the human wild type proteinase 3 sequence of SEQ ID NO: 7 is not considered to count towards to a proteinase 3 fragment.

In contrast to TALEN or Zinc finger nucleases, Cas enzymes can be programmed with a guide RNA. Preferably, in the context of the invention, the guide RNA is a single guide RNA.

In a preferred embodiment, the gene editor comprises a Cas protein, preferably, Cas9 protein, and a guide RNA selected from the group comprising SEQ ID NO: 1 (GCGGCCGUCAGCACGAAGCU), SEQ ID NO: 2 (UGACGGCCGCGCACUGCCUG) and SEQ ID NO: 3 (CGGCCGCUCACAUGUCCCGC). Guide RNAs can be chemically modified, e.g., by 2'-O-Methyl (2'-O-Me) modifications at the 3' and 5' ends of the sgRNA and 3' end phosphorothioate (PTO) linkages. The inventors have surprisingly found that gene editing based on SEQ ID NO: 1 is significantly superior to gene editing with the other tested guide RNAs. Therefore, the invention provides a gene editing compound comprising a Cas protein, preferably, a Cas9 protein, and a guide RNA, wherein the guide RNA has SEQ ID NO: 1.

However, guide RNAs of SEQ ID NO: 2 or 3 are also suitable for the use in the present invention. All of them significantly reduce expression of proteinase 3. Further guide RNAs can be prepared by the skilled person.

For example, further guide RNAs that can be advantageously used in combination with Cas9 or variants thereof such as Cas9-NG target the protospacer sequences provided in the table below to introduce a stop codon. A guide RNA targeting a protospacer preferably comprises a reverse complement of said protospacer sequence and a scaffold sequence mediating interaction with the Cas protein.

| **Protospacer sequence (without PAM) (SEQ ID NO)** | **PAM** |
|---|---|
| GCTGCCCGAGCTGCGGAGAT (8) | CG |
| TGCCCGAGCTGCGGAGATCG (9) | TGG |
| ACGAGGCGCAGCCACACTCC (10) | CGG |
| CCTGCAGATGCGGGGGAACC (11) | CGG |
| AGACCCCAGCGCCTGGTGAA (12) | CG |
| ACCCCAGCGCCTGGTGAACG (13) | TGG |
| GCGGACGCAGGAGCCCACCC (14) | AG |
| GCCCACCCAGCAGCACTTCT (15) | CGG |
| CCCACCCAGCAGCACTTCTC (16) | GG |
| CACCCAGCAGCACTTCTCGG (17) | TGG |
| CCTCATCCAGGTGGGCGGGC (18) | AGG |
| CACAGTCCAGCTGCCACAGC (19) | AGG |
| CTGCCACAGCAGGACCAGCC (20) | AG |
| CAGGACCAGCCAGTGCCCCA (21) | CGG |
| CGGCACCCAGTGCCTGGCCA (22) | TGG |
| GCGGCCCCAGCCCATGGCCA (23) | GG |
| CGCGGCCCCAGCCCATGGCC (24) | AGG |
| CCCCCAGCCCAGGTCCTGCA (25) | GG |
| CAGGTCCTGCAGGAGCTCAA (26) | TG |
| ATCCAAGGAATAGACTCCTT (27) | CG |
| ACATCCCCAGATCACGAAGG (28) | AG |
| GGCACATCCCCAGATCACGA (29) | AGG |
| CGGATCCAGTCCACGTAGAG (30) | GG |
| ACGGATCCAGTCCACGTAGA (31) | GGG |

Exemplary guide RNAs that can be used in combination with AsCas12a target the following protospacers: CCTGCAGCCTGGGGGCTCCCTGA, TGAACAACTACGACGCGGAGAAC, TTCTCCGCGTCGTAGTTGTTCAG, CACTTTCGTCCCTCGCCGCAAGG, GTCCCTCGCCGCAAGGCCGGCAT, CCCTGACTTCTTCACGCGGGTAG, CCTGACTTCTTCACGCGGGTAGC, GAGCCAGGCTCGGGGTCCCGGCC, GACAGAAGCAGCTCTTCCCCGAA, TTGAGGTCACGGTGGGGTGAGGT, AACGTTTATTGAGGTCACGGTGG (SEQ ID NO: 32-42).

The gene editor preferably targets and modifies the coding sequence of the PR3 gene. A gene editor can also modify a regulatory region, e.g., a promotor, an enhancer or a splice site, as long as the modification leads to reduced expression of PR3. The regulatory region preferably is in the PR3 gene. Such regulatory region can however also be outside of the PR3 gene on the human genome, even on a different chromosome, as long as it modifies and/or influences the expression of the PR3 gene. Expression of PR3 should be specifically reduced, i.e., expression of other genes such as neutrophil elastase should not be significantly affected.

For example, guide RNAs targeting splice sequences may target the protospacer sequences specified in the table below and can be used with CBE or ABE enzymes as specified:

| **Exon** | **Splice Site** | **Protospacer (SEQ ID NO)** | **PAM** | **Enzyme** | **cDNA dis- ruption** | **CBE posi-tion** |
|---|---|---|---|---|---|---|
| **3** | acceptor | GGGGTCTGCGGGGGTGGGGT (44) | GGT | CBE | 761 | 6 |
| **5** | acceptor | TCTCCCTGGAGGACAGGGAC (45) | GGC | CBE | 383 | 6 |
| **3** | donor | GCCCACCTGGATGAGGAGAA (46) | CGT | CBE, ABE | 617 | 6 |
| **3** | acceptor | TGGGGTCTGCGGGGGTGGGG (47) | TGG | CBE | 761 | 7 |
| **5** | acceptor | GTCTCCCTGGAGGACAGGGA (48) | CGG | CBE | 383 | 7 |
| **2** | donor | CTCACATGTCCCGCAGGCAG (49) | TGC | CBE, ABE | 761 | 5 |
| **2** | donor | CGCTCACATGTCCCGCAGGC (50) | AGT | CBE, ABE | 761 | 7 |
| **1** | donor | CACTCACCGCTCAGCAGCAA (51) | GGC | CBE, ABE | 929 | 7 |
| **4** | acceptor | CTCAGCTGGGGCAGAAGGCA (52) | GGC | CBE | 617 | 6 |
| **3** | acceptor | GCTGGGGTCTGCGGGGGTGG (53) | GGT | CBE | 761 | 9 |
| **1** | donor | TCACTCACCGCTCAGCAGCA (54) | AGG | CBE, ABE | 929 | 8 |
| **2** | acceptor | CACCTGCAGGGGGGGAGTCC (55) | AGG | CBE | 929 | 4 |
| **3** | acceptor | CGCTGGGGTCTGCGGGGGTG (56) | GGG | CBE | 761 | 10 |
| **5** | acceptor | TGAGTCTCCCTGGAGGACAG (57) | GGA | CBE | 383 | 10 |
| **4** | donor | TTACTTACGAAGCAGATGCC (58) | GGC | CBE, ABE | 383 | 8 |
| **2** | acceptor | GGCAGCACCTGCAGGGGGGG (59) | AGT | CBE | 929 | 9 |
| **3** | donor | GCCCGCCCACCTGGATGAGG (60) | AGA | CBE, ABE | 617 | 10 |
| **2** | donor | GGCCGCTCACATGTCCCGCA (61) | GGC | CBE, ABE | 761 | 10 |
| **4** | donor | GTTACTTACGAAGCAGATGC (62) | CGG | CBE, ABE | 383 | 9 |
| **4** | acceptor | GCTCAGCTGGGGCAGAAGGC (63) | AGG | CBE | 617 | 7 |
| **2** | donor | CGGCCGCTCACATGTCCCGC (64) | AGG | CBE, ABE | 761 | 11 |
| **4** | acceptor | CAGCTGGGGCAGAAGGCAGG (65) | CGG | CBE | 617 | 4 |
| **4** | acceptor | GCTGCTCAGCTGGGGCAGAA (66) | GGC | CBE | 617 | 10 |
| **2** | acceptor | CCCCCTGCAGGTGCTGCCCG (67) | AGC | ABE | 929 | NA |
| **2** | acceptor | CCTGCAGGTGCTGCCCGAGC (68) | TGC | ABE | 929 | NA |
| **2** | acceptor | TGCAGGTGCTGCCCGAGCTG (69) | CGG | ABE | 929 | NA |
| **3** | acceptor | CCCCCGCAGACCCCAGCGCC (70) | TGG | ABE | 761 | NA |
| **3** | acceptor | CCCCGCAGACCCCAGCGCCT (71) | GGT | ABE | 761 | NA |
| **3** | acceptor | CCGCAGACCCCAGCGCCTGG (72) | TGA | ABE | 761 | NA |
| **4** | acceptor | CTGCCCCAGCTGAGCAGCCC (73) | AGC | ABE | 617 | NA |
| **5** | acceptor | CTGTCCTCCAGGGAGACTCA (74) | GGT | ABE | 383 | NA |
| **5** | acceptor | GTCCTCCAGGGAGACTCAGG (75) | TGG | ABE | 383 | NA |
| **5** | acceptor | TCCTCCAGGGAGACTCAGGT (76) | GGC | ABE | 383 | NA |

In a preferred embodiment, the target sequence of the guide RNA binds to the proteinase 3 DNA close to the start codon, i.e., in the first third of the reading frame, e.g., within 250, optionally, within 200 nucleotides from the start codon. The sequence encoding the N-terminal signal peptide, which is not present in the processed protein, comprises exon 1 and 5 aa of exon 2. It is followed by a sequence encoding a dipeptide which is also not comprised in the processed protein. Preferably, the guide RNA binds to a sequence encoding amino acids present in the processed protein. For example, the guide RNA can bind to the second exon. The inventors have shown that targeting the second exon, e.g., with a guide RNA that leads to a frameshift in said codon, e.g., SEQ ID NO: 1, significantly reduces and even abrogates the presence of proteinase 3 in neutrophils.

The decisive factor for selection of the guide RNA is the capability of the agent in reducing proteinase 3 expression, as described herein.

The gene editor can be provided to the cells on a protein level or on a nucleic acid level, particular, as mRNA. Provision of a protein (or, in the case of a Cas enzyme, e.g., Cas9, a ribonucleoprotein complex, RNP) is very straightforward, and therefore preferred.

However, nucleic acids may be easier and cheaper to generate on a larger scale, and they also allow for an easy and stable genetic modification of the cells. The gene editing compound can thus also be a nucleic acid encoding the gene editor. The nucleic acid may be DNA, e.g., in the form of an expression vector allowing for expression of the gene editor in the cell. It preferably is RNA, in particular, an mRNA. An mRNA is typically chemically modified to enhance stability, e.g., as known in the art. The nucleic acid, e.g., the RNA preferably encodes a Cas enzyme such as Cas9 and the guide RNA, preferably, of SEQ ID NO: 1.

In the context of Cas9, application as RNP is preferred. In the context of base editors or prime editors, mRNA is preferably used.

In the context of the invention, a gene editor is an enzyme that modifies the sequence of a gene, here, the proteinase 3 gene. Preferably, it modifies the coding sequence thereof.

Alternatively, the expression of proteinase 3 can also be reduced using an epigenetic editing compound targeting the proteinase 3 gene. Whereas gene editing involves changing the actual DNA sequence itself, epigenetic editing involves modifying other factors that influence DNA function. Epigenetic editing can be site-specific DNA methylation editing or histone editing. For example, genome homing proteins with engineered or naturally occurring nuclease functions for gene editing can be mutated and adapted into delivery systems. An epigenetic modifying enzyme or domain can be fused to the homing protein and local epigenetic modifications can be altered upon protein recruitment. Exceptionally for DNA methylation, the homing domain itself can be enough to interfere with normal epigenetic processes to lead to targeted epigenetic editing. The epigenetic editing compound can be an epigenetic editor or a nucleic acid (DNA or RNA, preferably, RNA) encoding the epigenetic editor. The epigenetic editor can employ the same mechanisms for genomic modification as the gene editing compound, i.e., it can also be a
i) a ribonucleoprotein complex of a Cas protein and a guide RNA,
ii) a Zinc finger protein or
iii) a Transcription activator-like effector nuclease (TALEN).

Again, the epigenetic editing compound preferably comprises a protein, most preferably, a ribonucleoprotein complex of a Cas protein, as defined above, and a guide RNA. As in base editing or prime editing, for epigenetic editing, a catalytically impaired Cas enzyme is used that will not introduce a double strand break. For example, a dCas9 comprising a D10A and H840A mutation can be used. For site-specific methylation, a DNMT3a catalytic domain can be fused with the dCas9 protein. dCas9-DNMT3a is capable of achieving targeted DNA methylation of a targeted region as specified by the present guide RNA. Similarly, dCas9 can be fused with the catalytic core of an acetyltransferase, such as human acyltransferase p300. dCas9-p300 successfully catalyzes targeted acetylation of histone H3 lysine 27. Alternatively, the dCas9 protein alone is sufficient to physically interfere with normal processes which maintain DNA methylation at the site to which it is targeted in dividing cells; this results in targeted DNA demethylation, which can be more specific than combination with epigenetic-modifying enzymes (https://en.wikipedia.org/wiki/Epigenome_editing). Expression of PR3 should be specifically reduced, i.e., expression of other genes such as neutrophil elastase should not be significantly affected. In a preferred embodiment, the epigenetic editor, e.g., a Cas-based epigenetic editor, targets the promotor of the PR3 gene, e.g., modifying its methylation, and thereby specifically reduces expression of PR3 in the cell and cells derived therefrom. It can also target an enhancer, a splice site or another regulatory region or the PR3 gene.

While the genetic modification should be sufficiently stable to allow for reduction of the proteinase 3 expression in neutrophils derived from CD34+ HSPC modified with the agent of the invention, it is not essential that the proteinase 3 gene is modified in the cell's genome. Alternatively, expression can also be reduced or abolished at the mRNA level.

Accordingly, in another embodiment of the invention, the agent is an expression vector encoding an inhibitory oligonucleotide capable of selectively binding to a target nucleic acid sequence in an mRNA encoding proteinase 3, wherein the inhibitory oligonucleotide is selected from the group comprising an RNA molecule capable of inducing RNAi and an antisense oligonucleotide (ASO), preferably, an RNA molecule capable of inducing RNAi, e.g., shRNA.

In the context of the present invention, the term "oligonucleotide" is understood to refer to a short polymeric sequence of RNA or DNA nucleotides. An "inhibitory oligonucleotide" is herein understood as a type of oligonucleotide that is capable of post-transcriptionally reducing or even completely preventing the expression of the gene of interest into a protein by selectively binding to a target sequence within an mRNA expressed from the gene of interest, a process commonly referred to as gene "knockdown". Different types of inhibitory oligonucleotides are well-known in the art and include RNA molecules capable of inducing RNA interference (RNAi) such as microRNAs, small interfering RNAs or short hairpin RNAs (shRNAs) as well as antisense oligonucleotides (ASOs). Inhibitory oligonucleotides, in the context of the invention, may e.g. have a minimum length of 6 nucleotides (nt) and a maximum length of about 100 nt. However, typical inhibitory oligonucleotides consist of about 12-25 nt.

An inhibitory oligonucleotide according to the invention is considered capable of selectively binding to the target sequence when it can at least partially hybridize to the target sequence. In the context of the invention, hybridization refers to a process wherein two single-stranded nucleic acid molecules anneal to each other via complementary Watson-Crick base pairing. Moreover, an inhibitory oligonucleotide is considered able to "target" the proteinase 3 gene, when said oligonucleotide is able to selectively bind, i.e., to "hybridize" to a target mRNA encoding proteinase 3 via complementary Watson-Crick base pairing as defined herein.

The inhibitory oligonucleotide consists of, or comprises a sequence stretch that is complementary to any part of the target mRNA sequence and can hybridize to it, and that comprises 6-100, e.g. 12-25 nucleotides. In one embodiment, this stretch of the inhibitory oligonucleotide is 100% complementary i.e., hybridizes over its entire length to the target mRNA sequence, wherein each base of the guide sequence hybridizes to a corresponding complementary base within the target mRNA sequence. In such a scenario, the inhibitory oligonucleotide molecule is highly specific to its target sequence and thus typically does not bind to other sequences within a given sample.

However, in some embodiments, at least 1, e.g., at least 2, at least 3, at least 4, or at least 5 bases of the stretch of the inhibitory oligonucleotide that mediates binding to the target sequence can have mismatches with their corresponding target mRNA sequence, i.e., the sequence does not bind with 100% complementarity to its corresponding target sequence. Accordingly, the sequence stretch of the inhibitory oligonucleotide that mediates binding to the target sequence of the inhibitory oligonucleotide according to the invention may have a nucleic acid sequence that is merely 75-99%, e.g., 75%, 80%, 85%, 90%, 95% or 99% complementary to its target sequence. In such a scenario, the inhibitory oligonucleotide molecule still exhibits a very high specificity to its target sequence, while also allowing the recognition of variants thereof. This can be advantageous because the inhibitory oligonucleotide may then, e.g., be able to tolerate to some extent differences in the nucleic acid sequence of the proteinase 3 gene among human populations.

Optionally, the complementary sequence of the inhibitory oligonucleotide of the invention hybridizes to the target sequence with only a relatively short stretch of its sequence. For example, naturally occurring microRNAs (miRNAs) generally do not hybridize to a target RNA over their entire length. Rather, miRNAs only bind to their target mRNA with a short stretch of 6 to 8 nucleotides (nt) at their 5' end (the so-called seed sequence). Accordingly, the complementary sequence of an inhibitory oligonucleotide according to the invention may already be considered selective for a target sequence within the meaning of the present invention when it is complementary to at least a seed region of at least 5 to 15, e.g., of at least 5, at least 6, at least 7, at least 8, at least 9, at least 10, at least 11, at least 12, at least 13, at least 14, or at least 15 nucleotides of its respective target.

In a preferred embodiment, the inhibitory oligonucleotide is an RNA molecule capable of inducing a mechanism known as RNA interference (RNAi). RNAi is a natural and conserved molecular mechanism for post-transcriptional regulation of gene expression. In brief, RNAi involves the processing of double-stranded (ds) RNAs into shorter double stranded fragments of usually 20 to 25 base pairs (bp) inside a cell. Afterwards, the antisense strand of this dsRNA duplex, which is also referred to as guide strand, is incorporated into a multi-protein complex known as RNA-induced silencing complex (RISC). After association, this complex is recruited to a target mRNA molecule of interest via complementary base-pairing between the guide strand and a target sequence within said mRNA molecule, thereby blocking the translation of the mRNA transcript into a polypeptide. In instances where the antisense strand binds to its target sequence with near perfect complementarity, RNAi promotes the cleavage and irreversible degradation of the target RNA. Thus, RNAi can modulate both the stability and translational efficacy of mRNAs.

RNA molecules capable of inducing RNAi are also referred to herein as "RNAi-triggers". RNAi triggers can be of natural origin due to transcription of endogenous genes and further processing of the resulting primary transcripts to yield effective dsRNA duplexes. For instance, miRNAs are single-stranded, non-coding RNAs that are transcribed from endogenous miRNA-genes in the genome. It is estimated that hundreds of distinct and evolutionary conserved miRNAs modulate the expression of more than 60% of all human protein-coding genes within virtually every biomolecular pathway. Physiologically, miRNAs are generated from longer endogenous precursor transcripts (pri-miRNA), that form hairpin structures and are processed through two distinct endonucleolytic cleavage steps in the nucleus (via the enzyme Drosha) and cytoplasm (via the enzyme Dicer) into short RNA duplexes with a length of 20-23, e.g., 22 base pairs (bp) and a dinucleotide 3' overhang at both ends. Based on its thermodynamic properties defined by its sequence composition, the antisense strand of these duplexes is incorporated into RISC to guide the complex to a complementary target RNA.

siRNAs rely on a similar effector pathway as miRNAs but do not derive from endogenously transcribed hairpin structures. Instead, siRNAs can be either produced inside the cells from exogenous longer double stranded RNA fragments derived, e.g., from viral dsRNA, or they may be artificially designed and synthesized *in vitro* prior to their administration into cells.

Mature siRNAs typically have a length of 20 to 25 nt, preferably of 21 nt and, similar to miRNAs, rely on RNAi to prevent target RNAs from being translated into protein. However, whereas naturally occurring miRNAs typically silence tens to hundreds of genes by binding to the 3'UTR regions of the corresponding mRNAs guided by their 8 base pair "seed region" followed by repression of translation, synthetic siRNAs typically exhibit a higher specificity, often binding to their target sequences with up to 100% sequence complementarity, thereby inducing cleavage and degradation of their target mRNA via the RISC complex before translation.

Short hairpin RNAs (shRNA) constitute an alternative to siRNA-based therapeutics. shRNAs are artificially designed single stranded RNA molecules that exhibit a characteristic hairpin turn. shRNAs typically have a length of 50-70 nt and form a stem-loop structure consisting of a 19 to 29 bp region of double-strand RNA (the stem) bridged by a region of predominantly single-stranded RNA (the loop) and a dinucleotide 3' overhang. Once they are introduced into a cell, similarly to miRNAs, they are processed into short RNA duplexes that resemble siRNAs. One strand of this duplex is then incorporated into RISC to facilitate knock-down of a target gene. shRNAs can be encoded by an expression vector, such as a viral vector, which allows expression and processing of the shRNA construct inside a target cell.

shRNAs are normally transcribed by RNA polymerase III. However, the expression of exogenous shRNAs in cells can saturate the endogenous miRNA machinery, causing toxic effects to the cell. Moreover, imprecise processing of the stem loop structures of shRNAs may result in aberrant guide- and passenger-strand mediated off-target effects. To overcome these problems, shRNA may be provided as a "shRNAmiR", i.e., the shRNA is embedded within the backbone of a natural miRNA. The skilled person is aware of suitable miRNA backbones that can be used for the provision of shRNAs to cells, which may include e.g. the backbone of miR30a, miR223, miR144 or miR33 and they may be generated as described in e.g. ¹⁷. Such a shRNAmiR may also be referred to as an artificial miRNA or miRNA mimic. shRNAmiRs can be generated from Polymerase II promoters, enabling constitutive, tissue specific or inducible expression, as well as physiological expression levels.

The skilled person will be aware that neither siRNAs nor shRNAs must be fully complementary to their target sequences to effectively suppress protein synthesis, i.e., that some mismatches are admissible. Thus, if the siRNA antisense strand or the complementary sequence of the shRNA comprises one or two substitutions compared to the targeted mRNA sequence, it binds to its respective target sequence with imperfect complementarity but can nevertheless be a highly selective and potent RNAi trigger that specifically and effectively induces cleavage of the target RNA or inhibits its translation into protein. Multiple studies (e.g. ^{18,19}) suggest that mismatches affecting positions 4 -12 in siRNAs can reduce RNAi efficacy, whereas mismatches at positions 1, 2, 3, 18 and 19, in particular at positions 1 and 19, appear to affect RNAi efficacy only weakly or not at all ¹⁹. Thus, inhibitory oligonucleotide preferably does not comprise mismatches in positions 4-12 of the hybridizing stretch of nucleic acids.

In some embodiments, the inhibitory oligonucleotide of the invention is an siRNA. An siRNA is often provided as an RNA duplex of 20 to 27 base pairs (bp) consisting of a sense and a complementary antisense strand with characteristic 1 or 2 nt single-stranded overhangs on both sites. The antisense strand of the siRNA duplex serves as the guide strand, which is incorporated into RISC to target the complex to its respective target sequence to induce RNAi-mediated gene knock-down. The inhibitory oligonucleotide may also be only one strand of an siRNA duplex, i.e. it may only be the antisense strand comprising or consisting of a nucleic acid sequence having at least 90%, preferably at least 95%, more preferably 100% sequence identity to a target sequence in proteinase 3 mRNA.

In alternative embodiments, the RNA molecule capable of inducing RNAi may be an shRNA, whereas, optionally, a nucleic acid sequence having at least 90%, preferably at least 95%, most preferably 100% sequence identity to the target sequence in proteinase 3 mRNA forms a part of the stem of said shRNA. Accordingly, when the RNA molecule is an shRNA, it necessarily comprises additional nucleotides besides the complementary sequence having, e.g., at least 90%, preferably at least 95%, most preferably 100% sequence identity to the target sequence in proteinase 3 mRNA to facilitate proper folding of the RNA molecule into the stem loop structure of an shRNA.

Preferably, the shRNA is embedded into a miRNA backbone, also referred to as miRNA framework, as described herein, e.g., the framework of miR30a, miR223, miR144 or miR33. Accordingly, the RNA molecule may also be an shRNAmiR.

In another embodiment of the invention, the inhibitory oligonucleotide capable of selectively binding to the target nucleic acid sequence is not an RNA molecule capable of inducing RNAi but an antisense oligonucleotide (ASO). ASOs are artificial single-stranded oligonucleotides of about 12-25 nt, more typically of 15-22 nt length that target RNAs via complementary base pairing. They are usually made of DNA. ASOs can also consist of RNA.

ASOs exert their effects by two main mechanisms. On the one hand, ASOs can hybridize to their target RNAs to form DNA-RNA hybrids, which are recognized by RNase H, an endonuclease that triggers cleavage of the RNA strand of the DNA-RNA duplex. On the other hand, binding of ASOs to functional cis-acting elements within the target RNA may sterically block access of the cellular machinery to the RNA to, e.g., prevent translation or interfere with the splicing of the RNA.

The therapeutic use of ASOs is commonly referred to as antisense therapy.

In the context of the invention, the inhibitory nucleic acid is provided to the cell, typically, to a CD34+ HSPC, in the form of an expression vector, i.e., a vector configured to express the inhibitory oligonucleotide in situ, in particular, in a neutrophil derived from the HSPC, from a nucleic acid encoding said inhibitory oligonucleotide. The vector may be a minicircle, a plasmid, a cosmid, a phage, a virus or an artificial chromosome. Optionally, the expression vector is a viral vector, e.g., an adenoviral vector, a lentiviral vector or an adeno-associated viral (AAV) vector, in particular, an AAV vector. The expression vector can also be a transposon (e.g., in the form of a minicircle), which is typically transferred into the cell together with a transposase capable of mediating transposition of said transposon in protein or in nucleic acid form. The transposase can be, e.g., Sleeping Beauty, e.g., SB100X or a derivative thereof.

The expression vector comprises an expression cassette, i.e. the necessary elements that permit transcription of a cargo nucleic acid sequence into the inhibitory oligonucleotide, e.g. the shRNA, such as a suitable promoter. In one embodiment, the expression vector may comprise a cargo sequence that encodes only a single inhibitory oligonucleotide, e.g. a single shRNA as disclosed herein. Alternatively, the expression vector may comprise a cargo sequence that encodes a plurality of different inhibitory oligonucleotides, e.g., any combination of shRNAs. This way, it is possible to express multiple different shRNAs with different target sequences from a single expression vector. A person skilled in the art will be able to design suitable viral vectors to comprise a cargo sequence encoding one or more inhibitory oligonucleotides, e.g. one or more shRNAs based on general knowledge and the prior art. For example, a stretch of cargo DNA, i.e., a transgene, encoding the one or more inhibitory oligonucleotides, e.g., one or more shRNAs may be operably linked to a suitable promoter and inserted into the viral vector of choice. Other cargo sequences can also be contained in the expression vector operably linked to the same or a different promotor, e.g., a suicide switch.

A transgene is considered to be "operably linked" to a promoter when said promoter is positioned in a correct functional location and/or orientation in relation to said transgene to control its transcription. The promoter used to express the one or more inhibitory oligonucleotides may be any constitutively active promoter known in the art, e.g., a Rous sarcoma virus (RSV) promoter, a human cytomegalovirus (CMV) promoter, an HIV promoter or a eukaryotic promoter such as e.g., EF1a, PGK1, UBC, or human beta actin. Alternatively, the promoter may be an inducible promoter that only drives expression of the one or more oligonucleotide molecules in the presence or absence of a certain stimulus. For example, the inducible promoter may be a Tet-regulated promoter, i.e., it may comprise a tetracycline response element (TRE) that can be bound by a tetracycline transactivator (tTA) protein in the presence of tetracycline or an analogue thereof, e.g., doxycycline. The promoter may also be a cell-specific promoter which only drives expression in a particular type of cell, e.g., in a hematopoietic stem cell and/or a cell derived therefrom, in particular, in a neutrophil. Preferably, the promotor is a constitutively active or a cell-specific promotor.

Optionally, the vector furthermore is a cell-specific vector capable of targeting the one or more oligonucleotide molecules, e.g. the one or more shRNAs or shRNAmiRs, to a cell or a tissue of interest, preferably, a hematopoietic stem cell. It can also be targeted to a cell derived from a hematopoietic stem cell such as a neutrophil. Accordingly, if the vector is a viral vector such as a lentiviral vector, the assembled viral vector is optionally pseudotyped, e.g., with one or more different viral envelope glycoproteins or a capsid to facilitate binding of the vector to the surface of the cell of interest. The term pseudotyping refers to the generation of viral vectors that carry foreign viral envelope glycoproteins on their surface or are encapsulated in a capsid of a different virus or viral serotype to specifically target only particular cell types of interest. The viral vector particles are usually assembled inside suitable packaging cells known in the art according to standard laboratory techniques before they are administered to the subject.

The expression vector used for delivering the inhibitory oligonucleotide, e.g. the shRNA, may also be a non-pathogenic, genetically modified bacterium or yeast comprising a prokaryotic or eukaryotic vector. The vector comprises a DNA molecule encoding the candidate oligonucleotide molecule operably linked to a promoter that controls expression of said candidate oligonucleotide molecule, as described above. The non-pathogenic bacterium is preferably invasive, i.e., it is capable of entering a host cell and may be derived, e.g., from a non-pathogenic strain of *Escherichia coli, Listeria, Yersinia, Rickettsia, Shigella, Salmonella, Legionella, Chlamydia, Brucella, Neisseria, Burkolderia, Bordetella, Borrelia, Coxiella, Mycobacterium, Helicobacter, Staphylococcus, Streptococcus, Vibrio, Lactobacillus, Porphyromonas, Treponema,* or *Bifidobacteriae.*

The prokaryotic or eukaryotic delivery vehicle may also be specifically designed for targeted delivery to a cell of interest by expressing a targeting moiety on its surface. The targeting moiety may be, e.g., a cell-specific antibody or ligand such as, e.g., a small peptide, polysaccharide or nucleic acid that can be bound by a receptor expressed on the surface of the cell of interest. In a preferred embodiment, the targeting ligand is an anti-CD34 antibody, e.g., an anti-CD34 nanobody, i.e., a single domain antibody.

The present invention also provides a method of reducing expression of proteinase 3 in a cell selected from a hematopoietic stem cell and a cell derived therefrom, comprising contacting the cell with the agent of the invention, optionally, *in vitro.* In particular, the invention provides a method of reducing expression of proteinase 3 in a neutrophil, comprising contacting a hematopoietic stem cell with the agent of the invention, optionally, *in vitro.* Similar, a method of preparing a cell selected from a hematopoietic stem cell and a cell derived therefrom (e.g., a neutrophil) having a reduced expression of proteinase 3 is provided, comprising contacting the cell - or a progenitor thereof (e.g., a HSPC) with the agent of the invention, optionally, *in vitro.* The cell, in particular, the neutrophil, has a reduced expression compared to a cell, in particular, a neutrophil derived from a HSPC that has not been contacted with the agent of the invention, preferably, reduced by at least 50%, as described herein.

Depending on the agent, the contacting can comprise any of various methods, e.g., electroporation, viral transduction, transformation with calcium phosphate, or contacting with vesicles such as liposomes or lipid nanoparticles, bacterial minicells or an extracellular vesicle, the vesicles comprising the agent of the invention, e.g., the gene editing compound. Suitable methods are known to the skilled person. Electroporation is a preferred method for *in vitro* contacting the cells. For *in vivo* contacting cells, vesicles such as lipid nanoparticles are preferred. For example, combinations of lipid nanoparticles and mRNA encoding a gene editor such as a Cas enzyme and guide RNA can mediate efficient *in vivo* engineering or HSPC, e.g., using nanoparticles targeted to CD34+ HSPC.

The cell used for the method of the invention preferably expresses neutrophil elastase, and is not modified to reduce or abrogate expression of neutrophil elastase at least, if it is to be used for therapeutic purposes.

The invention also provides a cell obtainable from the method of the invention, preferably, a hematopoietic stem cell (also designated CD34+ HSPC herein). The cell can also be a cell derived from a hematopoietic stem cell, e.g., a neutrophil.

Also provided is a cell in which the proteinase 3 gene is knocked out or modified to reduce expression of proteinase 3. The invention alternatively provides a cell expressing an inhibitory oligonucleotide capable of selectively binding to a target nucleic acid sequence in mRNA encoding proteinase 3. These cells are obtainable from the method of the invention. The cell is structurally modified by the method of the invention. For example, if the agent is an expression vector, the cell comprises said expression vector, optionally, in a form integrated into the genome. If the agent is a gene editing compound or an epigenetic editing compound, the genome of the cell is modified through gene editing or epigenetic editing, respectively. The signature left by the gene editing or epigenetic editing, e.g., a signature of InDel mutations in the proteinase 3 gene, is characteristic for the agent used. Cas-mediated editing can e.g. lead to a cell comprising a nucleic acid of SEQ ID NO: 4, if the sgRNA has SEQ ID NO: 1. A preferred cell of the invention comprises a nucleic acid comprising SEQ ID NO: 4.

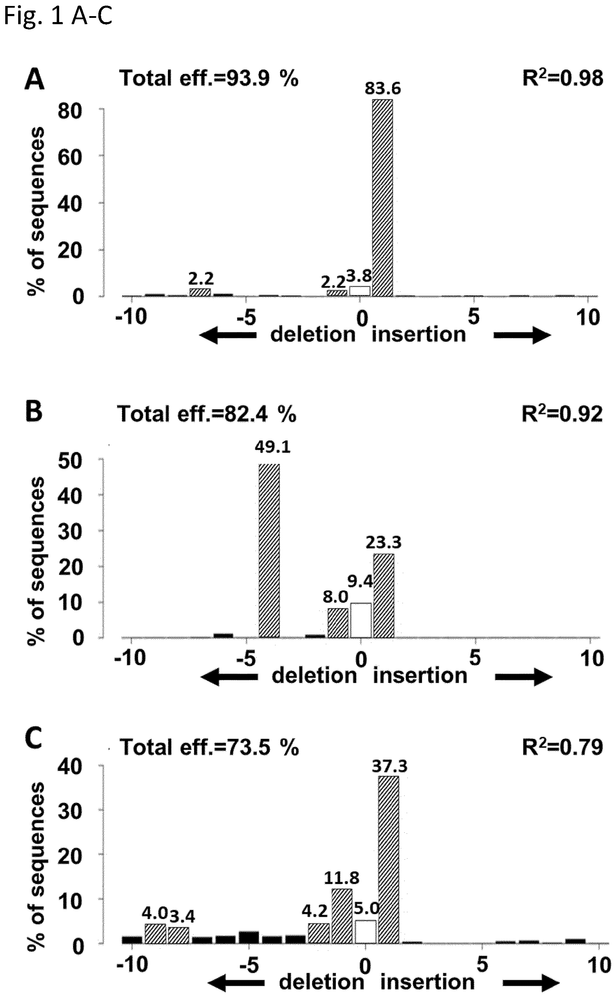

If the sgRNA has SEQ ID NO: 2 or 3, a mixture of sequences is generated, as also shown in Fig. 1. For example, if the sgRNA has SEQ ID NO: 2, SEQ ID NO: 5 is generated. In three positions of SEQ ID NO: 5, there is a high variability denoted by N, wherein N can be any of A, C, G or T. In a polyclonal population of cells obtainable by using of a gene editing compound with SEQ ID NO: 2 as sgRNA, N is a mixture of A, C, G and T. If the sgRNA has SEQ ID NO: 3, the generated sequence is CCTGCGNNNNNNNNNNNNNNNNN. Again, a high variability is denoted by N, wherein N can be any of A, C, G or T. In a polyclonal population of cells obtainable by using of a gene editing compound with SEQ ID NO: 3 as sgRNA, N is a mixture of A, C, G and T.

The invention also provides a population of cells comprising cells of the invention, wherein the population is a polyclonal population of cells, wherein, preferably, the population comprises cells with at least one modification, in particular at least one mutation, in the proteinase 3 gene that reduces proteinase 3 expression. Optionally, said cell population is obtainable from gene editing of the proteinase 3 gene of a respective cell population obtained from a human subject, e.g., by Cas-mediated gene editing, preferably, using a sgRNA of SEQ ID NO: 1. Advantageously, at least 50%, preferably, at least 70%, at least 80%, at least 90%, at least 95% or substantially all cells of the population carry the mutation in at least one, preferably, both proteinase 3 gene copies. The cells of the population are preferably CD34+ HSPC (e.g., at least 50% CD34+ HSPC, at least 70%, at least 80%, at least 90% or essentially all CD34+ HSPC). The inventors could show that highly pure cell populations can be produced using the methods disclosed herein, so that no selection of the produced cells is required before medical use thereof.

It is known in the art that there are subjects having a genetic disorder named Papillon-Lefèvre syndrome (PLS), also known as palmoplantar keratoderma with periodontitis. The disease leads, among other symptoms, to a severe destruction of periodontium that results in loss of most primary teeth by the age of 4 and most permanent teeth by age 14. Hyperkeratosis of palms and soles of feet appears in first few years of life. Destructions of periodontium follows almost immediately after the eruption of last molar tooth. Mutations in the cathepsin C gene (CTSC), located at human chromosome 11q14.1-q14.3, are the cause of PLS. The cells of the present invention do not carry a mutation in the cathepsin C gene that can lead to PLS. In contrast to the cells of PLS patients, the cell of the invention, in particular, the hematopoietic stem cell or the cell derived therefrom, expresses neutrophil elastase. Further, expression of proteinase 3 is stably reduced by at least 70 %, preferably, at least 90%, compared to a wild type hematopoietic stem cell or a cell derived from a wild type hematopoietic stem cell, in particular, a neutrophil. The wild type cell does not have a mutation in the cathepsin C gene, the neutrophil elastase gene or the proteinase 3 gene.

The cell of the invention can have further genetic modifications. For example, it can comprise a suicide switch. In another embodiment, it can be modified, e.g., gene-edited to allow for antigen-specific depletion of the rest of the entire hematopoietic system, including HSPCs, e.g., by an antibody drug conjugate targeting CD45. Accordingly, the CD45 gene may be modified, e.g., gene edited, for example, as disclosed in ²⁰. As a consequence, other HSPC can be depleted using the antibody drug conjugate targeting CD45, and the HSPC of the invention are not affected because they are not sensitive to said myeloablative therapy. Antibody-mediated myeloablation may replace classical myeloablative therapy.

In a preferred embodiment, the invention provides the cell of the invention, wherein the cell is a hematopoietic stem cell for use in treatment of a subject having a proteinase 3-ANCA-associated vasculitis. In one embodiment, the invention also provides the agent of the invention for use in treatment of a subject having a proteinase 3-ANCA-associated vasculitis.

For example, the cell of the invention, preferably, a cell population of the invention, is for use in treatment of a subject having a proteinase 3-ANCA-associated vasculitis. The treatment may e.g., comprise
a) mobilizing and isolating hematopoietic stem cells of the subject;
b) treating the subject with myeloablative therapy;
c) *in vitro* contacting the isolated hematopoietic stem cells with the agent of the invention to obtain modified hematopoietic stem cells
d) administering the modified hematopoietic stem cells to the subject.

Steps b and c are always performed after step a and, if the modified hematopoietic stem cells are sensitive to the myeloablative therapy, before step d. Steps b and c can however be carried out in parallel or in any order. If the modified hematopoietic stem cells are not sensitive to the myeloablative therapy, step b can also be carried out after step d.

The myeloablative therapy can be classical myeloablative therapy comprising treating the subject with cyclophosphamide and/or busulphan, e.g., as known in the art^{19,30}.

In a preferred embodiment, the agent is a gene editing compound, e.g., a gene editor such as a complex of Cas9 and a guide RNA, preferably, having SEQ ID NO: 1, and the modified hematopoietic stem cells are hematopoietic gene-edited stem cells. The *in vitro* contacting can be carried out, e.g., as described above, e.g., by electroporating the cell with the nucleoprotein complex. The modified HSPC can be administered to the subject without selection, as described herein. A selection step can also be performed. Selection can be positive selection, e.g. for expression of a marker that the cell is simultaneously engineered to express, or it can be negative selection, e.g., for lack of proteinase 3 expression. For this, no marker protein would be required. Before administration of the HSPC, they can be expanded, e.g., as known in the art ³⁰. Alternatively, they can be administered without prior expansion, e.g., without selection and without expansion. Myoablation and expansion can e.g., be performed as described ³⁰.

For example, 1-20 × 10⁶, preferably, at least 2x 10⁶, optionally, 3-17 × 10⁶ CD34+ cells per kilogram body weight HSPC can be administered to a patient, preferably, as a composition of the invention as defined herein ²¹. The HSPC are preferably administered intravenously in a composition suitable for intravenous administration, which may e.g., comprise a physiological NaCl solution or a buffer. HSPC of the invention may be frozen after modification and thawed before application.

Alternatively, the modification of the HSPC is not carried out *in vitro* but *in vivo,* wherein the treatment comprises administering the agent of the invention to the subject. For example, a viral vector or a lipid nanoparticle may be administered that comprises an agent of the invention, e.g., a nucleic acid (DNA or RNA, preferably, mRNA) encoding a gene editor or an epigenetic editor (preferably, a gene editor), e.g., comprising a Cas9 nucleoprotein complex which may for instance comprise the guide RNA having SEQ ID NO: 1. Said agent, e.g., said nanoparticle, may be targeted to CD34+ HSPC *in vivo* via a targeting agent, e.g., via an antibody to CD34 such as a single-domain antibody. The agent may also be a protein linked to such a targeting agent.

The subject treated according to the invention is a subject freshly diagnosed with AAV, in particular, with granulomatosis with polyangiitis, i.e., a patient. The patient can be freshly diagnosed. However, preferably, the patient is refractory to therapy or has a relapse of the disease. The patient can also receive the treatment of the invention after a first diagnosis and, optionally, after a first immunosuppressive therapy, to prevent a first or a further relapse.

In a preferred embodiment, the treatment comprises testing for the presence of proteinase 3-ANCA in a sample from the subject before further treatment steps are carried out, and administering the cell or agent to the subject if proteinase 3-ANCA are detected in the sample,

wherein said testing comprises contacting the sample with proteinase 3 and detecting binding of proteinase 3-ANCA to proteinase 3. For example, PR3 can be immobilized to a plate and a sample from a subject to be tested for the presence of PR3-ANCA, such as plasma, added. If PR3-ANCA are present in the sample, they bind to the PR3, after washing, binding can be detected with a secondary antibody, e.g., an anti-human IgG antibody linked to a detection agent such as horseradish peroxidase or luciferase.

Testing avoids treatment of subjects that will not profit from the treatment of the invention. Accordingly, the invention also provides a kit comprising proteinase 3 suitable for detecting proteinase 3-ANCA, e.g., immobilized to a solid support such as a plate, optionally, further comprising a secondary antibody for use in testing a subject for the presence of proteinase 3-ANCA in a sample from the subject, wherein, if proteinase 3-ANCA are detected in the sample, the cell or agent of the invention are administered to the subject. Such a kit can also comprise an agent of the invention.

Advantageously, the hematopoietic stem cells administered to the subject are autologous hematopoietic stem cells. It is one of the advantages of the invention that the treatment does not require, and preferably does not comprise any immunosuppressive treatment after administration of the cell or the agent.

Also disclosed is a method of treating a subject having proteinase 3-ANCA-associated vasculitis, comprising administering a cell of the invention or an agent of the invention to the subject, as described herein.

In the context of the present invention, the term "a" is intended to mean one or more, unless explicitly mentioned otherwise. For example, the treatment of the invention can also comprise treatment with two or more agents of the invention.

The present invention is further exemplified and illustrated by the following examples. These are not intended to be limiting to the invention. All literature cited is herewith fully incorporated herein.

### Legends

Fig. 1. TIDE analysis of the sanger sequencing comparison of three sgRNA for CRISPR-Cas9-mediated disruption of the PRTN3 gene in the region of interest. (A) sgRNA SEQ ID NO: 1 (B) sgRNA SEQ ID NO: 2 (C) sgRNA SEQ ID NO: 3. TIDE analysis (http://shinyapps.datacurators.nl/tide/), shaded bars P<0.001, black bars P>= 0.001.

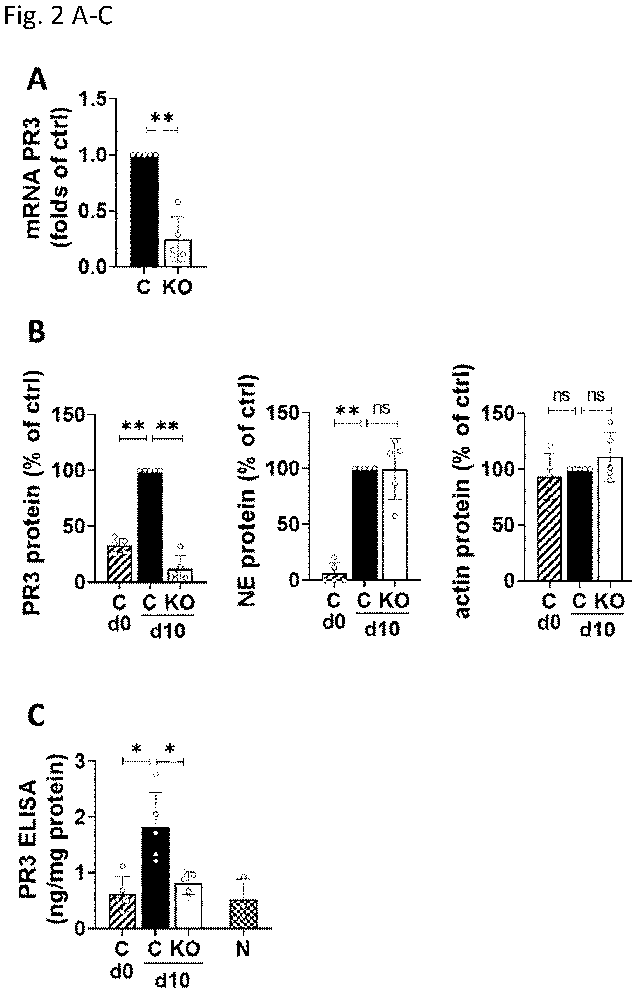

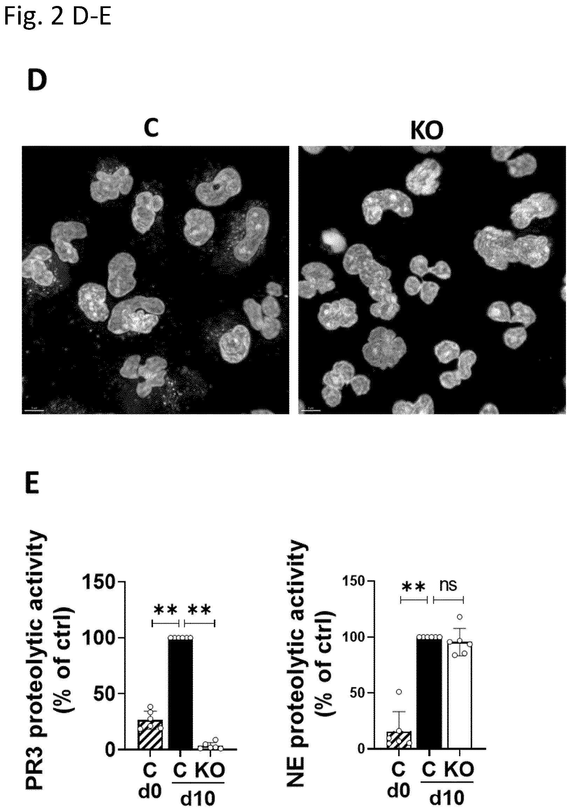

Fig. 2. (A) PR3 gene expression was analysed in PR3KO (*KO, white bars*)- and non-transfected Ctrl-HSPCs (*C*, *black bars*) by qRT-PCR at day 3 of differentiation (n=5). (B) PR3 protein was efficiently reduced in PR3KO as shown by immunoblotting at day 10 (*left panel*)*,* HNE (*middle panel*) and actin (*right panel*) served as controls. In addition, corresponding protein expression at day 0 is shown (*C*, *shaded bars*). Optical densities (OD) of the protein bands were quantified (n=5). (C) PR3 was assessed in PR3KO- and non-transfected Ctrl-HSPCs by ELISA (n=5). PR3-non-expressiing cells (*N, chequered bar*) served as additional control (n=3). (D) Superimposed confocal laser scanning microscopy images of PR3KO- and non-transfected Ctrl-HSPCs at day 10 of differentiation. PR3 staining is shown in cyan, DAPI nuclear staining in grey. Zeiss LSM, 63x oil objective, Image acquisition with Zeiss ZEN software (E) PR3-gene editing abolished PR3 but not HNE proteolytic activity as detected by specific FRET substrates (n=6). Individual results and mean ± SD are given. For statistical analysis, 1-way ANOVA with matched or repeated measures (RM) and following Sidak's post-hoc test was performed after normalization to non-transfected Ctrl-HSPCs. *P<0.05, **P<0.01

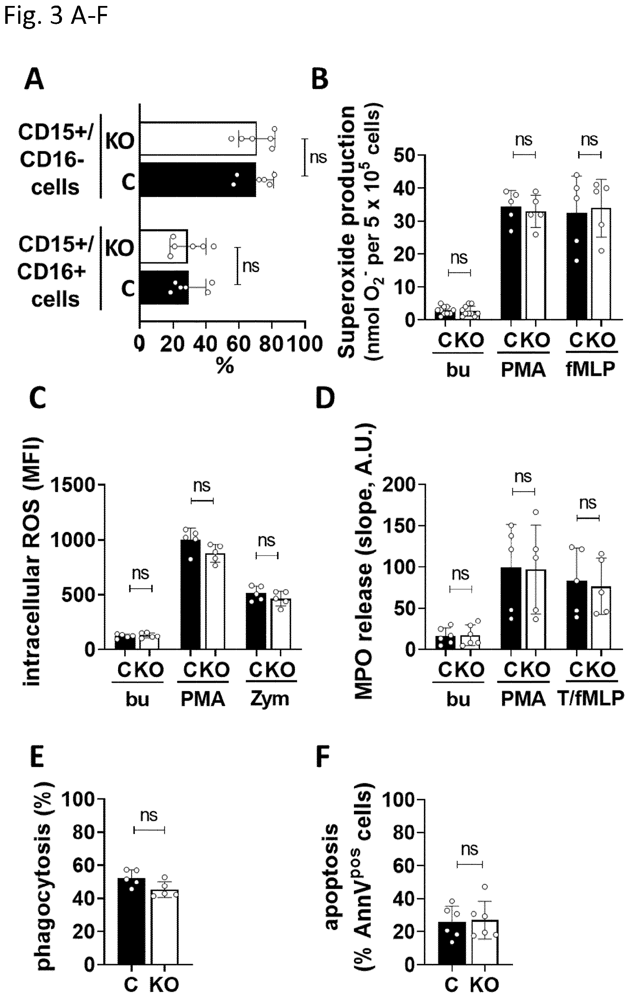

Fig.3. (A) FACS data showing the quantification of percentages of CD15+/CD16- and CD15+/CD16+ cells in PR3KO (*KO, white bars*)- and non-transfected Ctrl-HSPCs (*C*, *black bars*) (n=6). PR3 disruption does not affect receptor-independent (PMA) or receptor-dependent (fmlp, Zymosan (Zym), or TNF+fmlp respectively), mediated neutrophil functions such as (B) extracellular superoxide (O²⁻) release (control (bu), n=10; PMA and fmlp, n=5), (C) intracellular reactive oxygen species (ROS) generation (n=5), (D) degranulation, monitored by MPO enzymatic activity in cell-free supernatants (n=5), (E) phagocytosis, assessed by pH-sensitive pHrodo BioParticles. Phagocytic activity is represented as percent fluorescent positive cells (n=5), and (F) constitutive apoptosis after 24 hours, assessed by flow cytometry Annexin V staining (n=5). Individual data points, mean and SD are given. For statistical analysis, paired t-test (two-tailed) or repeated measures 1-way ANOVA with Sidak's post-hoc test was performed. *P<0.05, **P<0.01.

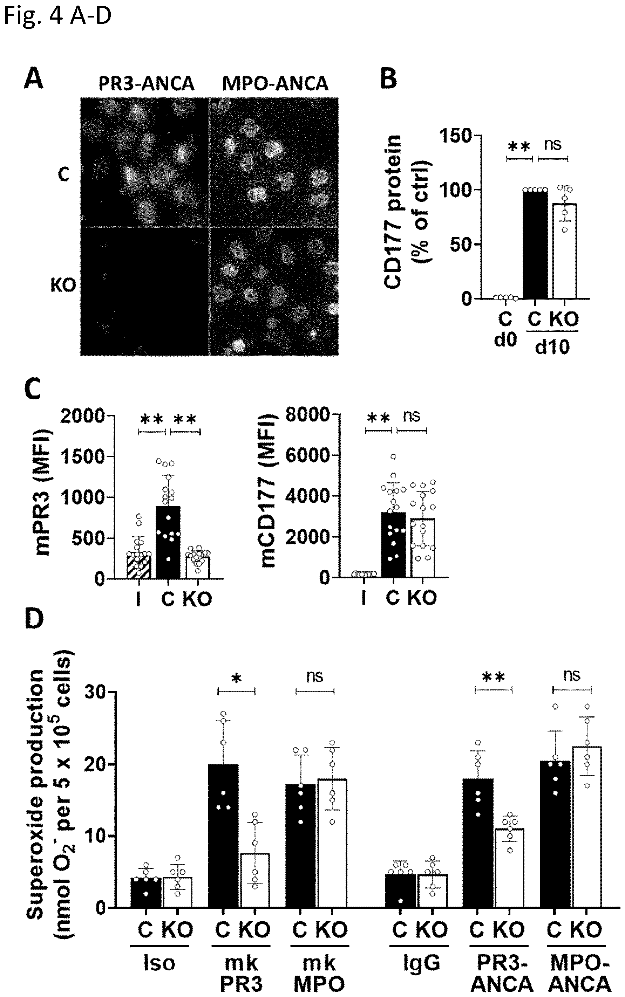

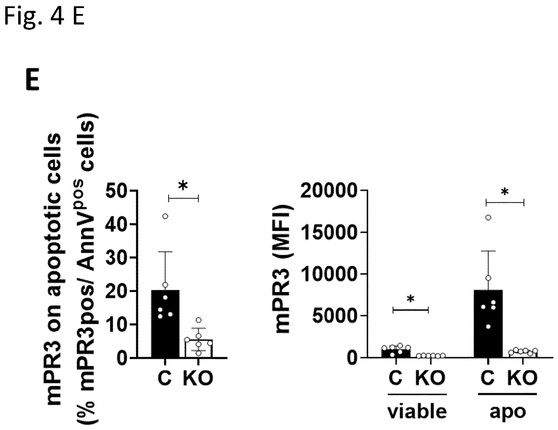

Fig.4. (A) Indirect immunofluorescence using PR3KO- and non-transfected Ctrl-HSPCs neutrophils and sera from patients with PR3- or MPO-ANCA positive AAV. Stainings for PR3- or MPO are shown grey. Leica DMI6000 B microscope, 63x glycerol objective. (B) Monitoring of protein levels of the PR3-receptor CD177 by immunoblotting. Optical densities (OD) of the protein bands were quantified (n=5). (C) Assessment of PR3 (*left panel*) and CD177 (*right panel*) on the cell surface of viable PR3KO- (*KO, white bars*) and non-transfected Ctrl-HSPCs (C, *black, bars*) by flow cytometry. Isotype stainings (I, *shaded bars*) are shown as controls (n=16). (D) Superoxide release by PR3KO- and non-transfected Ctrl-HSPCs differentiated for 10 days was assessed using monoclonal antibodies (mAbs) to PR3 and MPO, human PR3- and MPO-ANCA IgG by the ferricytochrome C reduction assay (n=6 independent experiments, each using 3 different ANCA preparations). (E) PR3KO- and non-transfected Ctrl-HSPCs were differentiated for 10 days. Next, cells were kept in RPMI medium for 20 hours. Constitutive apoptosis and membrane PR3 were monitored by Annexin V-/ PR3 flow cytometry staining. Quantification of the percentage of AnnV-/PR3 double positive cells (*left panel*) and of membtrane-PR3 amount on viable and apoptotic cells (*right panel*) (n=6). Individual results and mean ± SD are given. Paired t-test (two-tailed) or repeated measures 1-way ANOVA with Sidak's post-hoc test was performed. *P<0.05, **P<0.01.

### Sequences

| | | |
|---|---|---|
| SEQ ID NO: 1 | preferred gRNA | GCGGCCGUCAGCACGAAGCU |
| SEQ ID NO: 2 | gRNA | UGACGGCCGCGCACUGCCUG |
| SEQ ID NO: 3 | gRNA | CGGCCGCUCACAUGUCCCGC |
| SEQ ID NO: 4 | DNA sequence generated by gene editing with SEQ ID NO: 1, inserted base in bold, PAM in italics | *CCC*AGC**T**TTCGTGCTGACGGCCG |
| SEQ ID NO: 5 | DNA sequence generated by gene editing with SEQ ID NO: 2, mutated bases in bold, PAM initalics, N can be C, G, T or A | TGACGGCCGCGCACTGC**NNN***GC*A |
| | Frequent DNA sequence generated by gene editing with SEQ ID NO: 3 mutated bases in bold, PAM in italics, N can be C, G, T or A | *CCT*GCG**NNNNNNNNNNNNNNNNN** |
| SEQ ID NO: 7 | Human wild type PR3 sequence | |
| SEQ ID NO: 8-42, 44-76 | Protospacers targeted by exemplary gRNAs | |
| SEQ ID NO: 77 | Primer for gDNA PCR and PR3 sequencing | |
| SEQ ID NO: 78 | Primer for gDNA PCR | |

### EXAMPLES

### Methods

### sgRNA design

The sgRNA for targeting exon 2 of human *PRTN3* gene were designed as previously described²² using CRISPRscan (https://www.crisprscan.org/) with UCSC Genome Browser (human GRCh38/hg38). Potential sgRNA candidates were initially manually screened for low numbers of predicted off-targets using COSMID. Three chemically modified sgRNA (#1-3, SEQ ID NO: 1-3) were synthesized by Synthego (Redwood City, CA), incorporating 2'-O-Methyl (2'-O-Me) modifications at the 3' and 5' ends of the sgRNA and 3' end phosphorothioate (PTO) linkages to enhance their stability and ultimate gene editing efficacy.

### Cell culture and nucleofection

HEK293 cells were cultivated in Dulbecco's modified Eagle's medium high glucose supplemented with 10% FCS and penicillin/streptomycin. HEK293 cells were nucleofected at 70-80% confluence using the Amaxa^{™} 4D-Nucleofector X kit (Lonza, Basel, Switzerland). Cells were harvested, washed and resuspended in supplemented Nucleofector V Solution (2×10⁵ cells in 15µl). The solution was mixed with a ribonucleoprotein complex formed during an incubation of SpCas9 and sgRNA at 37°C for 10 min. For each nucleofection, 200,000 cells with 5µg SpCas9 and 6µg sgRNA in a mass ratio of 1:1.2 was used in a 20 µL reaction (16-well nucleofection cuvette). Nucleofection was performed using an Amaxa 4D Nucleofector (Lonza, Basel, Switzerland) with the pulse program CM-130. Afterward, 80 µL supplemented DMEM medium was added, and cells were plated in a 12 well plate. CD34⁺ hematopoietic stem and progenitor cells (HSPCs) were nucleofected using Amaxa^{™} P3 Primary cell 4D-Nucleofector X kit (Lonza). and supplemented Nucleofector Solution Set P3 as described above. Here, 400,000 cells with 5µg SpCas9 and 6µg sgRNA in a mass ratio of 1:1.2 was used in a 20 µL reaction for each nucleofection. The Nucleofector runs with the pulse program for human CD34+ cells (EO-100).

### gDNA sequencing

HEK293 were lysed in 1M Tris/ 0.5M sodium EDTA/ 1MNaCl and protein precipitated using 5M sodium perchlorate. Genomic DNA was isolated by chloroform/ isopropanol precipitation and sequencing performed from the PR3 PCR products (with Advantage GC 2 polymerase mix & PCR kit, Takara, Saint-Germain-en-Laye, France) using the primer listed in the table below. PCR products were purified from agarose gel using NucleoSpin Gel and PCR Clean-up kit (Macherey-Nagel GmbH & Co. KG, Düren, Germany). After purification, 50ng of the PCR products were subjected to DNA sequencing using BigDye Terminator v1.1 Cycle Sequencing kit from Thermo Fisher Scientific (Schwerte, Germany). Purification of the samples was done with ZR DNA Sequencing Clean-up kit (ZYMO Research, Freiburg, Germany). DNA sequencing was performed with a 48-capillary sequencer 3730xl Genetic Analyzer (Applied Biosystems/Thermo Fisher Scientific, Darmstadt, Germany).

### Primer for gDNA PCR and sequencing:

| Method | Primer name | Primer sequence (SEQ ID NO) |
|---|---|---|
| PCR of region of interest with genomic DNA | PRTN3-KO-For1 | GAGTCCTTCCCACCAGCCAG (77) |
| | PRTN3-KO-Rev1 | CTCCGAGCACCACGTTCACC (78) |
| Sanger sequencing | PRTN3-KO-For1 | GAGTCCTTCCCACCAGCCAG (77) |

### Differentiation of CD34⁺ stem and progenitor cells from umbilical cord blood into neutrophils

CD34⁺ hematopoietic stem and progenitor cells (HSPCs) were isolated from umbilical cord blood (Ethics vote EA4/024/18), expanded, and differentiated as described previously⁴. Briefly, cells were washed, stained, e.g. using CD34⁺ progenitor isolation kit (Miltenyi), and sorted on a LS column. Cell expansion was performed in stem span serum-free medium (Stem Cell Technologies, Vancouver, Canada) with penicillin/streptomycin, 100 ng/mL stem cell factor (SCF), 20 ng/mL TPO, and 50 ng/mL FLT3-L (PeproTech, London, United Kingdom). Neutrophil differentiation was done in RPMI with 10% FCS and 10 ng/mL G-CSF (PeproTech) for 10 days.

### Preparation of human neutrophils

Blood from healthy humans was obtained after written informed consent (Ethics vote EA4/025/18). Neutrophils were isolated from heparinized whole blood by dextran sedimentation and density gradient centrifugation as described⁴. Cells were >95% neutrophils as determined by morphological analysis and >99% viable by Trypan blue dye exclusion.

### SDS-PAGE and immunoblotting analysis

Cell lysis, SDS-PAGE, and immunoblot were performed as described". In brief, HSPCs or differentiated neutrophils were lysed, mixed with loading buffer, separated on a 12% SDS-PAGE, blotted onto polyvinylidene difluoride (PVDF) membrane, developed with the indicated antibodies, and visualized by an ECL detection system (Super Signal West Dura Extended Duration substrate, Fisher Scientific, Schwerte, Germany) on a Chemi Only Imager (VWR International, Darmstadt, Germany). The monoclonal rabbit anti-human PR3 (1:4,000, Abcam, Cambridge, UK), polyclonal rabbit anti-NE (1:1,000, Abcam), polyclonal goat anti-CD177 (1:1,000, R+D systems, Minneapolis, MN), and polyclonal rabbit anti-actin (1:2,000, Cell Signaling Europe, Frankfurt/Main, Germany) were used together with corresponding secondary horseradish peroxidase conjugated antibodies (1:1,000).

### Fluorescence resonance energy transfer (FRET) and NSP-specific proteolytic activity

PR3-specific proteolytic activity was measured as described previously ²³. Briefly, cells were lysed on ice, soluble fractions were separated from cell debris by centrifugation. Five to 30 µg cell lysates in 150 µl HEPES buffer containing 0.02% lauryl maltoside (LM) were incubated with the human PR3-specific FRET substrate 2-Abz-VAD-(nor)V-ADYQ-EDA-Dnp or NE-specific FRET substrate 2-Abz-APEEIMRRQ-EDA-Dnp (20 µM) final) Fluorescence was measured by plate reader (excitation 320 nm, emission 420 nm, SpectraMax M5, Molecular Devices, CA) and the Vmax is reported.

### Flow cytometry to assess neutrophil mPR3 and mCD177

For mPR3 and mCD177 double staining, cells were incubated with 5 µg/ml Alexa488 conjugated anti-PR3 (clone 43-8-3) or corresponding Alexa488-isotype for 15 min on ice. After washing CD177 was stained with anti-CD177-pacific blue (PB) and mouse IgG-PB mAbs were used as isotype control (Exbio, Vestec, Czech Republic). 20,000 events per sample were collected using a BD FACS Calibur or a BD FACS CANTO II and analyzed with FlowJo software (TreeStar, Ashland, OR).

### Differentiation Marker FACS

Cells were resuspended in phosphate-buffered saline (PBS) for subsequent staining and flow cytometry. Fluorochromeconjugated antibodies were used directed to CD45 (2D1; BD Biosciences), CD11b (M1/70; eBioscience, San Diego, CA), CD15 (W6D3, Biolegends, San Diego, CA) and CD16 (3G8, Biolegends). All cell populations were gated as live CD45 cells after exclusion of doublets and further characterized with their respective markers. Samples were acquired using an FACS CANTO II flow cytometer with BD Q13 FACSdiva software (BD Biosciences). Data were analyzed Q14 using FlowJo software (Treestar, Ashland, OR).

### Preparation of human IgG

Normal- and ANCA-IgG were prepared from healthy subjects and patients with active MPO- and PR3-ANCA disease using a High-Trap-protein-G column in an Äkta-FPLC system (Cytiva Europe GmbH, Freiburg, Germany).

### ANCA testing by indirect immunofluorescence

Neutrophils were centrifuged on glass slides using a Cytospin Hettich Universal device (Hettich GmbH, Tuttlingen, Germany) and permeabilized in ice-cold 99% ethanol. Slides were incubated with PR3- or MPO-ANCA patient sera (1:10 diluted in PBS) for 60 min at room temperature. After washing, cells were stained with Alexa488-conjugated anti-human IgG (1:250, Molecular Probes, Eugene, Oregon, USA) for 60 min, washed with PBS, and covered with fluoromount (Southern Biotech, Birmingham, AL, USA). Fluorescence images were acquired using a Leica Microscope (Leica DM16000 B, Wetzlar, Germany) with a 40x objective.

### Measurement of superoxide release

Superoxide was measured using superoxide dismutase (SOD)-inhibitable ferricytochrome c (final concentration 50 µM) reduction as described previously²⁴. Briefly, 5x 10⁵ cells/ml were pretreated with cytochalasin B (5 µg/ml, 15 min), primed with TNFalpha (2 ng/ml, 15 min) before stimulating antibodies (mAbs 5 µg/ml, or 75 µg/ml purified IgGs) were added. Assay was performed in 96-well plates at 37 °C, and the absorption of samples with and without SOD (300 U/ml) was scanned repetitively at 550 nm using a Microplate Reader (Molecular Devices) and 45 min results are reported.

### Measurement of intracellular ROS production

Prewarmed neutrophils (1 ×10⁷/ml in HBSS) were loaded with CM-H₂DCFDA (1 µM), Thermo Fischer Scientific, Waltham, MA) for 15 min at 37 °C. Cells (5x 10⁵) were incubated with the stimuli in a total assay volume of 100 µl for 45 min. Data were collected from 20,000 cells per sample using a BD FACS CANTO II and analyzed with FlowJo software (TreeStar, Ashland, OR). The shift of green fluorescence in the FL-1 mode was determined, and the mean fluorescence intensity (MFI, representing the amount of generated hydrogen peroxide) is reported.

### Phagocytosis of pHrodo E. coli bioparticles

Cells (5x 10⁵) were incubated with pHrodo Green E. coli bioparticles (100 µg/ml, Thermo Fischer Scientific) at 37 °C in a total assay volume of 100 µl for 120 min. Data were collected from 20,000 cells per sample using a BD FACS CANTO II and analyzed with FlowJo software (TreeStar, Ashland, OR).

### Apoptosis assessment by annexin V and 7-AAD staining

2x 10⁶ differentiated neutrophils were incubated in RPMI medium with 10% FCS for up to 72h. Phosphatidylserine exposure was determined by Annexin V-FITC (BD Biosciences, San Jose, CA) and necrosis by 7-AAD (Merck, Darmstadt, Germany) staining.

### Measurement of MPO degranulation

Cells (5x 10⁵) were pretreated with 5 µg/ml cytochalsin B 200µl on ice for 15 min, prior receptor independent stimulation with 25ng/ml phorbol ester (PMA) or receptor-dependent stimulation with 2ng/ml TNFalpha/ 2x 10⁻⁶ M N-formyl-methionyl-leucyl-phenylalanine (fmlp) at 37 °C for further 30 min. After centrifugation cell-free supernatant was assessed for MPO enzymatic activity with MPO substrate 2,2'-azino-bis(3-ethylbenzothiazoline-6-sulfonic acid (ABTS, Merck, Darmstadt, Germany), Absorption was measured by plate reader (wavelength 405 nm, VersaMax, Molecular Devices, CA) and the Vmax is reported.

### Results

### Identification of a highly efficient CRISPR-Cas9 nuclease to disrupt PRTN3

To identify a suitable single-guide RNA (sgRNA) to disrupt PR3 protein by Cas9-mediated gene editing, we designed three sgRNAs (#1-3) targeting exon 2 in the *PRTN3* gene with low predicted off-target activity and compared their ability to introduce disruptive indels. To this end, HEK293 cells were transfected with a ribonucleoprotein (RNP) complex consisting of one of the sgRNAs and Cas9 protein by electroporation. Sanger sequencing was performed to compare the indel rate and profile of the three sgRNAs using TIDE²⁵ (Fig. 1). The sgRNA-#1 (Gg18NGG-61) showed the highest indel frequency of 93,9%, predominantly resulting in a +1bp insertion, inducing a frame shift in the *PRTN3* gene (Fig. 1). Based on these results, we selected sgRNA-#1 for further experiments evaluating the consequence of *PRTN3* gene editing for PR3 protein abundance in neutrophil-differentiated human CD34+ HSPCs.

### CRISPR-Cas9 gene editing effectively reduces PR3 protein and abrogates PR3 enzymatic activity in neutrophil-differentiated human CD34+ HSPC

The RNP complex of Cas9 protein and PR3-specific sgRNA-#1 was electroporated into cytokine-activated, proliferating human CD34+ HSPCs ^{22, 26}. Transfected and non-transfected control HSPCs were then differentiated into neutrophils. At day 10 of differentiation, PR3^{KO}- and non-transfected Ctrl-HSPCs were assessed for PR3 mRNA, PR3 protein, and proteolytic PR3 activity, respectively. Quantitative PCR demonstrates significant down-regulation of PR3 transcription (Fig. 2A) and immunoblotting significantly reduced PR3 but not HNE protein abundance in PR3^{KO}-neutrophils (Fig. 2B). Human neutrophil elastase (HNE) was included as a control protein because it is encoded within 3 kb downstream of the *PRTN3* gene on chromosome 19 and is, together with PR3, Cathepsin G and NSP4, a member of the neutrophil serine protease (NSP) family. Effective PR3 protein reduction in PR3^{KO}-neutrophils was confirmed using a PR3-specific ELISA (Figure 2C) and high-resolution microscopy (Fig. 2D). In addition, the proteolytic PR3 but not HNE activity was abrogated in PR3^{KO} neutrophils as demonstrated by a protease-specific FRET assays (Figure 2E). These experiments indicate successful disruption of the PR3 protein by CRISPR-Cas9 gene editing of human CD34⁺ HSPCs.

### Disruption of PR3 does not compromise differentiation and physiological inflammatory responses of neutrophil-differentiated HSPCs

First, we asked whether disruption of PR3, also named myeloblastin, affects neutrophil differentiation of human CD34⁺ HSPCs. CD34⁺ HSPCs from umbilical cord blood were expanded, gene edited when indicated, and subsequently differentiated into neutrophils. We observed similar percentages of differentiated neutrophils at the end of the differentiation period by flow cytometry (Fig. 3A).

Next, we investigated whether disrupting PR3 protein and enzymatic activity would compromise neutrophil functions that are important for host defense. Neutrophils derived from control HSPCs and from PR3 gene edited HSPCs were treated with receptor-independent and -dependent stimuli and assessed for extracellular superoxide release (Fig. 3B), intracellular ROS production (Fig. 3C), degranulation (Fig. 3D), phagocytosis of E. coli particles (Fig. 3E) and constitutive apoptosis (Fig. 3F), respectively. We found no significant differences in the activation responses or in constitutive apoptosis between PR3^{wt}- and PR3^{KO}-neutrophils in either of the assays. These results suggest that PR3 is dispensable for a variety of inflammatory responses and for apoptosis that is pivotal to inflammation resolution.

### Disruption of the PR3 autoantigen reduces binding of ANCA from PR3-AAV patients and PR3-ANCA induced neutrophil functions

In clinical medicine, the presence of ANCA in patient serum is evaluated by an indirect immunofluorescence (IIF) test on ethanol-permeabilized normal human neutrophils. Patients harboring autoantibodies to PR3 show positive cytoplasmic staining (c-ANCA) whereas those with autoantibodies to MPO show perinuclear staining (p-ANCA). We used sera from AAV patients and performed an ANCA IIF test using either neutrophils derived from control or PR3 gene-edited HSPCs. Typical c-ANCA staining was observed when PR3^{wt}- but not PR3^{KO}-neutrophils were incubated with sera from PR3-AAV patients (Fig. 4A). Thus, patient sera containing polyclonal PR3-ANCA yielded a negative immunofluorescence after specific disruption of the PR3 autoantigen. This finding underscores the efficacy of PR3 disruption and, in addition, strengthens the notion that PR3 is the single autoantigen in PR3-AAV. Sera from MPO-AAV patients were used as a control and yielded the typical p-ANCA staining pattern on both PR3^{wt}- and PR3^{KO}-neutrophils.

To activate neutrophils, PR3-ANCA bind to PR3 that is presented on the neutrophil-surface, largely by the subset-restricted expression of the CD177 receptor. Consequently, CD177^{neg}/membrane-PR3^{low} and CD177^{pos}/membrane-PR3^{high} neutrophil subsets exist in humans. We evaluated the possibility that residual PR3 amounts were present on the surface of viable neutrophils despite PR3 gene disruption. First, we investigated whether PR3 gene disruption affected CD177 protein. We observed similar CD177 protein amounts in PR3^{wt}- and PR3^{KO}-neutrophils by immunoblotting (Fig. 4B). Next, we assessed the presentation of CD177 and PR3 on the cell surface of viable PR3^{wt}- and PR3^{KO}-neutrophils by double-staining and flow cytometry. We found strongly reduced PR3, but not CD177, on the surface of PR3^{KO}- compared to PR3^{wt}-neutrophils (Fig. 4C).

Next, we evaluated the activation of PR3^{wt}- and PR3^{KO}-neutrophils in response to mabs to PR3 and to PR3-ANCA IgG from different AAV patients. Using a robust respiratory burst assay and appropriate controls, we observed strong superoxide generation in PR3^{wt}-neutrophils treated with mAbs to PR3 and PR3-ANCA IgG, respectively (Fig. 4D). This activation response was significantly reduced when PR3^{KO}-neutrophils were treated with these antibody preparations. In contrast, a mAbto MPO and MPO-ANCA IgG also induced superoxide release, but this response was similar in PR3^{wt}- and PR3^{KO}-neutrophils.

### No significant indel formation at predicted off-target in PR3-KO HSPCs and differentiated neutrophils

For the clinical translation of the PR3-KO approach to treat AAV patients, gene editing must be both effective and safe. The off-target activity of CRISPR-Cas9 poses a potential risk for the PR3-KO approach. To address this issue, we employed three orthogonal in silico prediction tools: Cas-OFFinder, COSMID, and CCTop. These tools identified 50 potential off-targets for the PRTN3-specific sgRNA#1. All nominated off-target sites contained at least three mismatches to the target sequence, as the gRNA was specifically designed for high specificity to minimize high-risk off-target sites.

We performed next-generation sequencing using a custom pooled rhAmp-seq panel to measure indel formation at the predicted off-target sites in HSPCs two days after electroporation but before starting differentiation and, in addition, in HSPC-derived neutrophils at day 10 of differentiation. Comparison of edited and control-electroporated HSPCs demonstrated high indel rates at the on-target, but no significant indels at any of the nominated off-target sites. These results emphasize the high degree of precision of the selected sgRNA-Cas9 nuclease and support the further clinical translation of the PR3-KO approach for treatment of AAV patients.

### Discussion

We explored gene editing to disrupt the PR3 autoantigen as a potential treatment for PR3-AAV patients. Our study revealed several new findings. First, gene editing of human HSPCs using CRISPR-Cas9 and an appropriate sgRNA effectively disrupts the PR3 protein. Second, PR3 was dispensable for neutrophil differentiation and a variety of neutrophil defense functions. Third, PR3 gene disruption strongly reduced the PR3 autoantigen and thus PR3-ANCA binding to and subsequent activation of neutrophils. Moreover, ANCA-independent PR3-controlled inflammatory efferocytosis, and proteolytic anti-inflammatory AnnexinA1 reduction were diminished.

PR3 is a member of the neutrophil serine protease (NSPs) family together with human neutrophil elastase (HNE), cathepsin G (CG), and the recently characterized neutrophil serine protease 4 (NSP4). NSPs are produced as zymogens and proteolytically activated by Cathepsin C (CatC), also known as dipeptidyl peptidase 1 (DPP1; EC 3.4.14.1). CatC loss-of-function mutations lead to Papillon-Lefèvre syndrome (PLS, OMIM: 245000), a rare autosomal recessive disease. All NSPs share several substrates but have also distinct cleavage sites and contexts reflected by their corresponding iceLogos. Several effects of active NSPs contribute to inflammation, and inhibition of NSP activity by CatC inhibitors is currently explored in neutrophil-mediated inflammatory diseases. PR3 is a unique NSP acting not only as a serine protease, but also as the disease-driving autoantigen in PR3-AAV.

Our CRISPR-Cas9 *PRTN3* gene editing approach specifically disrupted the PR3 protein. HNE protein derived from the *HNE* gene that resides in close proximity to *PRTN3* served as a control NSP and was not affected. Importantly, the lack of PR3 protein, also named myeloblastin, did not compromise HSPC differentiation into a neutrophil phenotype indicated by similar expression of the neutrophil lineage and maturation marker CD15 and CD16, respectively. Our findings obtained with human HSPCs agrees with the normal neutrophil numbers, their maturation, and turn-over reported in mice overexpressing PR3. Moreover, we showed that PR3-depleted neutrophils showed similar activation responses compared to control neutrophils and no change in constitutive apoptosis. Thus, our findings support the notion that PR3 is dispensable for neutrophil differentiation and that PR3-disrupted neutrophils have no defect in neutrophil defense functions such as respiratory burst, degranulation, phagocytosis. This makes it possible to use PR3 gene editing in patients.

AAV patients harbor either ANCA to PR3 or MPO that are exclusively expressed by neutrophils and monocytes. ANCA binding to and activation of these myeloid cells with subsequent vascular inflammation and injury is central to the AAV pathogenesis. Current standard treatments include glucocorticoids, cytotoxic drugs, B cell-depleting antibodies, and complement inhibition to reduce inflammation and suppress the patients' autoimmune response. With these drugs, disease remission is achieved in approximately 75% of AAV patients after 12 months. However, a recent meta-analysis from randomized controlled trials showed that 58% of the patients relapsed after a median of 3.6 years and that infection was the leading cause of death. Thus, more effective treatments with less adverse effects are needed. We reported previously that MPO- and PR3-AAV patients showed increased PR3 protein pools in plasma and neutrophils, respectively. Importantly, only in PR3-AAV patients increased PR3 amounts correlated with systemic inflammation, kidney injury, and PR3-ANCA titer underscoring the PR3 antigen-driven disease process. Deleting the PR3 autoantigen provides an advantageous treatment strategy compared to current protocols that suppress the inflammatory and autoimmune response. We show that PR3 gene-editing effectively reduced the PR3 autoantigen amount. This effect will reduce PR3 antigen-driven adaptive immune cell responses. In addition, we found strongly diminished PR3 protein in and on viable neutrophils. Consequently, less PR3-ANCA will bind to these gene-edited neutrophils resulting in less neutrophil activation. Moreover, although PR3 disruption did not affect the rate of constitutive neutrophil apoptosis, PR3 was strongly reduced on the surface of apoptotic neutrophils. PR3 on apoptotic neutrophils was implicated in inflammatory efferocytosis of these cells by macrophages and, in the presence of PR3-ANCA, in Th17 cell polarization. Conceivably, PR3 reduction on both viable and apoptotic neutrophils will co-operate in reducing PR3-AAV activity. Because animal models for PR3-AAV are lacking, PR3 gene editing cannot be evaluated in preclinical disease models.

CRISPR-Cas gene editing in HPSCs is a promising clinical platform for autologous gene therapy of severe diseases with long-term efficacy. Precise gene editing does not affect clonality and long-term engraftment in preclinical models and also in patients with β-haemoglobinopathies^{7,8}. Our genotoxicity data attests a high precision of the PR3-specific sgRNA-Cas9 complex. Previous studies have demonstrated that use of orthogonal *in silico* prediction algorithms is a reliable method to identify off-targets ²⁷. In our study, we used well-established *in silico* prediction tools and, subsequently, evaluated all nominated sites with next-generation amplicon sequencing without evidence of significant indel formation at potential off-target sites.

Autologous HSPC transplantation (aHSCPT) is used to reset autoimmunity in a variety of autoimmune diseases. However, the reoccurrence of autoreactive T- and B cells after initial successful depletion threatens long-term remission. Reduction of PR3 expression in neutrophils, e.g., by ex *vivo* gene editing to disrupt the autoantigen thus enables improved patient outcome in PR3-AAV, a disease featuring a single autoantigen. Autoantigen disruption by gene editing of autologous HSPCs together with the concomitant myeloablative conditioning can achieve the ultimate goal of drug-free remission. Our data establishing efficacy and specificity of PR3 gene editing in human HSPCs without detecting undesired effects provides proof-of concept for clinical application in PR3-AAV patients.

### References

1. Shochet, L. & Kitching, A.R. Animal models of vasculitis. Curr Opin Rheumatol 34, 10-17 (2022).
2. Korkmaz, B., Kuhl, A., Bayat, B., Santoso, S. & Jenne, D.E. A hydrophobic patch on proteinase 3, the target of autoantibodies in Wegener granulomatosis, mediates membrane binding via NB1 receptors. J Biol Chem 283, 35976-35982 (2008).
3. Loughner, C.L. et al. Organization, evolution and functions of the human and mouse Ly6/uPAR family genes. Hum Genomics 10, 10 (2016).
4. von Vietinghoff, S. et al. N B1 mediates surface expression of the ANCA antigen proteinase 3 on human neutrophils. Blood 109, 4487-4493 (2007).
5. Eulenberg-Gustavus, C., Bahring, S., Maass, P.G., Luft, F.C. & Kettritz, R. Gene silencing and a novel monoallelic expression pattern in distinct CD177 neutrophil subsets. J Exp Med 214, 2089-2101 (2017).
6. Lodka, D. et al. CD19-targeting CAR T cells protect from ANCA-induced acute kidney injury. Ann Rheum Dis 83, 499-507 (2024).
7. Frangoul, H. et al. Exagamglogene Autotemcel for Severe Sickle Cell Disease. N Engl J Med 390, 1649-1662 (2024).
8. Locatelli, F. et al. Exagamglogene Autotemcel for Transfusion-Dependent beta-Thalassemia. N Engl J Med 390, 1663-1676 (2024).
9. Longhurst, H.J. et al. CRISPR-Cas9 In Vivo Gene Editing of KLKB1 for Hereditary Angioedema. N Engl J Med 390, 432-441 (2024).
10. Gillmore, J.D. et al. CRISPR-Cas9 In Vivo Gene Editing for Transthyretin Amyloidosis. N Engl J Med 385, 493-502 (2021).
11. Tebas, P. et al. Gene editing of CCR5 in autologous CD4 T cells of persons infected with HIV. N Engl J Med 370, 901-910 (2014).
12. Vakulskas, C.A. et al. A high-fidelity Cas9 mutant delivered as a ribonucleoprotein complex enables efficient gene editing in human hematopoietic stem and progenitor cells. Nat Med 24, 1216-1224 (2018).
13. Komor, A.C., Kim, Y.B., Packer, M.S., Zuris, J.A. & Liu, D.R. Programmable editing of a target base in genomic DNA without double-stranded DNA cleavage. Nature 533, 420-424 (2016).
14. Rees, H.A. & Liu, D.R. Base editing: precision chemistry on the genome and transcriptome of living cells. Nat Rev Genet 19, 770-788 (2018).
15. Walton, R.T., Christie, K.A., Whittaker, M.N. & Kleinstiver, B.P. Unconstrained genome targeting with near-PAMless engineered CRISPR-Cas9 variants. Science 368, 290-296 (2020).
16. Anzalone, A.V. et al. Search-and-replace genome editing without double-strand breaks or donor DNA. Nature 576, 149-157 (2019).
17. Adams, F.F. et al. An optimized lentiviral vector system for conditional RNAi and efficient cloning of microRNA embedded short hairpin RNA libraries. Biomaterials 139, 102-115 (2017).
18. Du, Q., Thonberg, H., Wang, J., Wahlestedt, C. & Liang, Z. A systematic analysis of the silencing effects of an active siRNA at all single-nucleotide mismatched target sites. Nucleic Acids Res 33, 1671-1677 (2005).
19. Ahmed, F. & Raghava, G.P. Designing of highly effective complementary and mismatch siRNAs for silencing a gene. PLoS One 6, e23443 (2011).
20. Garaude, S. et al. Selective haematological cancer eradication with preserved haematopoiesis. Nature 630, 728-735 (2024).
21. Frangoul, H. et al. CRISPR-Cas9 Gene Editing for Sickle Cell Disease and β-Thalassemia. N Engl J Med 384, 252-260 (2021).
22. Gundry, M.C. et al. Highly Efficient Genome Editing of Murine and Human Hematopoietic Progenitor Cells by CRISPR/Cas9. Cell Rep 17, 1453-1461 (2016).
23. Jerke, U. et al. Neutrophil serine proteases exert proteolytic activity on endothelial cells. Kidney Int 88, 764-775 (2015).
24. Schreiber, A., Luft, F.C. & Kettritz, R. Membrane proteinase 3 expression and ANCA-induced neutrophil activation. Kidney Int 65, 2172-2183 (2004).
25. Brinkman, E.K., Chen, T., Amendola, M. & van Steensel, B. Easy quantitative assessment of genome editing by sequence trace decomposition. Nucleic Acids Res 42, e168 (2014).
26. Bak, R.O., Dever, D.P. & Porteus, M.H. CRISPR/Cas9 genome editing in human hematopoietic stem cells. Nat Protoc 13, 358-376 (2018).
27. Cromer, M.K. et al. Comparative analysis of CRISPR off-target discovery tools following ex vivo editing of CD34(+) hematopoietic stem and progenitor cells. Mol Ther 31, 1074-1087 (2023).
28. WO 2020/008466 A1
29. www.lempo-tx.com
30. Sharma et al N Engl J Med 2023;389:820-32

## Claims

1. An agent for reducing expression of proteinase 3 in a cell, wherein the agent is
a) a gene editing compound targeting the proteinase 3 gene, or
b) an epigenetic editing compound targeting the proteinase 3 gene, or
c) an expression vector encoding an inhibitory oligonucleotide capable of selectively binding to a target nucleic acid sequence in mRNA encoding proteinase 3, wherein the inhibitory oligonucleotide is selected from the group comprising an RNA molecule capable of inducing RNAi and an antisense oligonucleotide (ASO).

2. The agent of claim 1, wherein the agent is a gene editing compound targeting the proteinase 3 gene selected from
- a gene editor comprising
i) a ribonucleoprotein complex of a Cas protein and a guide RNA,
ii) a Zinc finger protein or
iii) a Transcription activator-like effector nuclease (TALEN), and
- a nucleic acid encoding the gene editor.

3. The agent of claim 2, wherein the gene editor is a Cas9 protein and a guide RNA selected from the group comprising SEQ ID NO: 1, SEQ ID NO: 2 and SEQ ID NO: 3.

4. The agent of claim 3, wherein the guide RNA has SEQ ID NO: 1.

5. The agent of claim 1, wherein the agent is an epigenetic editing compound targeting the proteinase 3 gene.

6. The agent of claim 1, wherein the agent is an expression vector encoding an inhibitory oligonucleotide capable of selectively binding to a target nucleic acid sequence in an mRNA encoding proteinase 3, wherein the inhibitory oligonucleotide is selected from the group consisting of an RNA molecule capable of inducing RNAi and an antisense oligonucleotide (ASO), preferably, an RNA molecule capable of inducing RNAi.

7. A method of reducing expression of proteinase 3 in a cell selected from a hematopoietic stem cell and a cell derived therefrom, comprising contacting the cell with the agent of any of claims 1-6, optionally, in vitro.

8. A cell obtainable from the method of claim 7, preferably, a hematopoietic stem cell.

9. A cell wherein the proteinase 3 gene is knocked out or modified to reduce expression of proteinase 3 or a cell expressing an inhibitory oligonucleotide capable of selectively binding to a target nucleic acid sequence in mRNA encoding proteinase 3, wherein the cell optionally is a cell of claim 8.

10. The cell of any of claims 8 or 9 comprising a nucleic acid of any of SEQ ID NO: 4-6, preferably, SEQ ID NO: 4.

11. The cell of any of claims 8-10, wherein the hematopoietic stem cell or a cell derived therefrom expresses neutrophil elastase and wherein expression of proteinase 3 is stably reduced by at least 70 % compared to a wild type hematopoietic stem cell or a cell derived from a wild type hematopoietic stem cell.

12. The cell of any of claims 8-11, wherein the cell is a hematopoietic stem cell, or the agent of any of claims 1-6 for use in treatment of a subject having a proteinase 3-ANCA-associated vasculitis.

13. The cell or agent for use of claim 12, wherein the treatment comprises
a) mobilizing and isolating hematopoietic stem cells of the subject;
b) treating the subject with myeloablative therapy;
c) in vitro contacting the isolated hematopoietic stem cells with the agent of any of claims 1-6 to obtain modified hematopoietic stem cells,
preferably, wherein the agent is the agent of any of claims 2-4 and the modified hematopoietic stem cells are hematopoietic gene-edited stem cells;
d) administering the modified hematopoietic stem cells to the subject.

14. The cell or agent for use of any of claims 12 or 13, wherein the treatment comprises testing for the presence of proteinase 3-ANCA in a sample from the subject before further treatment steps are carried out, and administering the cell or agent to the subject if proteinase 3-ANCA are detected in the sample,
wherein said testing comprises contacting the sample with proteinase 3 and detecting binding of proteinase 3-ANCA to proteinase 3.

15. The cell or agent for use of any of claims 12-14, wherein the hematopoietic stem cells are autologous hematopoietic stem cells, and the treatment does not comprise any immunosuppressive treatment after administration of the cell or the agent.
